# EUROPEAN PATENT APPLICATION

(11) **EP 1 666 883 A1**
(43) Date of publication of application: **07.06.2006**
(21) Application number: 05014631.5
(22) Date of filing: 03.02.2003
(51) Int. Cl.: G01N 33/68

(54) **Diagnostic and monitoring methods for bone loss**

(30) Priority: 08.02.2002 US 355255 P
(62) Divisional of application: 03704519.2
(71) Applicant: F. HOFFMANN-LA ROCHE AG, 4070 Basel (CH); The Government of the United States d.b.a. The Department of Veterans Affairs, Washington, DC 20420 (US); Oregon Health & Science University, Portland, OR 97201-3098 (US)
(72) Inventor: Allard, John David, Milpitas, CA 95305 (US); Klein, Robert Frederick, Portland, Oregon 97225 (US); Peltz, Gary Allen, Redwood City, CA 94062 (US)
(74) Representative: Witte, Hubert

(57) **Abstract**

Methods of treating and preventing bone loss and/or enhancing bone formation are disclosed. The methods utilize 15-lipoxygenase inhibitors. These molecules can be delivered alone or in combination with agents which inhibit bone resorption or additional agents that regulate calcium resorption from bone or enhances bone accumulation. The invention additionally provides methods of diagnosing a predisposition to bone loss.

SEQUENCE LISTING

<110> F. Hoffmann-La Roche AG

<120> Method of treating and preventing bone loss

<130> Case 21150

<150> US60/355255

<151> 2002-02-08

<160> 2

<170> PatentIn version 3.1

<210> 1

<211> 7864

<212> DNA

<213> Mus musculus

<220>

<221> Ensembl gene ENSMUSG00000018924

<222> (1)..(7864)

<223>

<220>

<221> position 70929619 in mouse genome

<222> (1)..(1)

<223>

<220>

<221> position 70937482 in mouse genome

<222>  (7864)..(7864)

<223>

<400>  1

tctttgcaac attttattta aaataattag gtccctgttc ttttctatca ctagcccaaa      60

acatctttta ttgttgctat tagactctat taaggcttga gatctatttg attcatagac     120

gatgtgtaac aatgtatatt atcacccttt tgtgttcccg gggtgctttt cccaagcatg     180

gctattattt accctatggg gaaggaaaag cagaaaggat gaatgaaaag tggcactctg     240

ggtaacgcat ccaaagcaaa atgtgttcac tacagcagac tatggaaagc gggctcttgg     300

aaaagggttc cacactggca gagggcgggg ctcaagcaca gggatgcact tgagtggtct     360

tgattaaata acaaatcgag aggggggatg gtcatatggc cacgctgttt tctaccaggc     420

tgggccgcag gtactcataa ggtatgtcca agctcttgtt acgaatctca atttccttat     480

ccaaggcagc cagctcctct ctgaacttct tcagcacagc tttggcctca gggtttggga     540

agtgttcctc tgaatgctgg cccaggggca cctggaaggc aggtgggaaa acagcctaag     600

cctgtgacct ccgccagttt tggggtgcag agtttcctct ggaccctgaa tgattgattg     660

actctcacca taacagcctg gcgtctgccc aggagccaaa cgacatttat ctggagagta     720

gactgattag gattgggcag cgtcgccatc agcttctcca tcgtcgcgtc cttggtttta     780

ggtggtggca gccgcatggt gcagggtgca ttaggaaccc agtagaacca atccagctgg     840

agagacagtg atctagggtc aaggctcgcc cttggccccc atgctcacat tcccctcccc     900

cactgtactc cccaaattcc cagctgcctc cctctgggaa gtacctggcc aagatggatg     960

gaagagtgct gcgcagtgca tgtgaagatg cacatggtga tgaagtggca agcctgagcc    1020

cgggactgga gggaggtggg gaagcctacg agaggtgtga acggttagcg tgtgacgtca    1080

ccagaaccac tagacgggag gcctggagag gagagggaga ggcgagaagg acctcccagg    1140

accttgtgta agctggtatg agagcgggga tggtgaggga cgagaggagc cctggcacct    1200

atgtcataca taccccatcc ccaaggctca gggatcattg tcaaagggat gacagaaaaa    1260

acttttgaga gttttcatga agatttaaat gcttctggga gagtccatgg aagcacagag    1320

ggttctggag acgtctcaga actcaggtct tagttctcag gccagggatt cttattacct 1380

ctgtcctggg caccttgcaa cccaatctca gtgatctctt gacaccagct ctgcagttca 1440

tagtcatcct gaacagcttg gtcggtcttg tagtagagat cgaacattcc caccacgtac 1500

ctgtcaccaa agggtcaggg caagctgagg ccctctgctc cctgctccat gtggctgagt 1560

catagtgtcc acacccaacc aagccaagga acagacctcc tattctcctg gtcttctcca 1620

cccacaccgg cacacaaacc cgtcctgcgg ctctgttgac ctcaccgatt catgacttgc 1680

cagagctgca gggcatcttt agcatagaag caagtgtcaa tatccaggag tcctcgctca 1740

gccaagtcat cagggggaca caatgagctg taggtcagga aggctccagc ttgcttgaga 1800

agatccaggt ggcctcctcc acctgtgctc atcacctaaa ggaccaaggg caggtggttt 1860

tcactctaat caagctgccc tgaagggaga ggacagagtg aacctagagc ccacttctat 1920

ccccatacct tgtcaaaaaa gcctctctct gagatcaggt cgctcctggc ccggacattg 1980

atttccatgg tgtagagtag gtgaggaact agaagctagg agaagaaaag ggatgggtaa 2040

gtcagaagga ctctatgaca ggtccctgta gcaagcaggt ttgacaaggg agaagcgaag 2100

ctcactccaa gggaaaacgt ggccgtgagg ctctgtggcc ctcgattctg ataagtgctg 2160

gttttctgcc tgactggggc cacaagagtc aaatcactgc cttctgacaa tctctttcat 2220

tccctgtgtt gtcttttcaa cagtctttct tgatcaccca aaagaaactc acatggcact 2280

gttgtgattc tctaagatct caccacagcc aacatgtcga gtataatcac tactggggtt 2340

tctgttgtta tttttgtttt gctttgtttg tttttttttt cctcttactg gagataaaaa 2400

aaaaatcccc caaaactaga tgttaacaca ggcatctaga ctaggtctgt gtagagacat 2460

ccaataccaa tttcgatgga tgaactcttt gctctgattg actagagtaa accaggtcac 2520

tcttgttgct ctaggtcatc gtttcagaac tccccaaaga ctgctgtaag gaatccgttg 2580

tgtaatttgc atggtcccct ctttaagtca cgtgcaccta ctctggtact gtatgtatat 2640

ctatgttctg tacataagat tagaatcttt acaccctttg ctgaggaatc acctgtatat 2700

gcccccttgg ggattagtgc cattgctgac aatgtagagg acagcaggtc acactccctt 2760

tctctctccg ttgttcatct catctcttct catcatcctg aaccctgcct gagatgccac 2820

tgagagaccg tctcagaggc aagagtgtgt agcaagccaa gagatgtaga cagaacaaaa 2880

aaaaacacaa aaaacaaaaa acaaacaaac aaaaaaaaac cgaataaggt gttaccttaa 2940

aaacagggtg cacggaaggc aggcacctca tggtggccac agcaaagacc tcagccacca 3000

agtgtcccct cagaagatga gcctgtagct catgaagctg taagtctgag cttcggaccc 3060

agcatttggc caggagccag tccattgggg gatccaaggg cgtgaaaatc ggcggtgggg 3120

tagacccagt tttgggcagt tcgagctgga gtaaaacaac agtgtgaaga aagggctgga 3180

tggagatctt cgtggcaacg gctcagcgat ccctacttcc tccatcccac acgtgtgcag 3240

ccatcttggg ctctctctca tctttcaaca tcacctctcc acaaagatct gaggctctgg 3300

gtttcaacta atagaggatt ttgtatgtgt ccccattact tcaaaactgg caggaccaaa 3360

gcctacctgg gtgcctaccc ctctgcactt gccctgtgtc tgaagaaacc tgcctgagtc 3420

ccacttttcc tgactccact ccaggccagt agattctccc tctgctctcc ctcctctcca 3480

gctccatggc tacttactac tcacggagag cccaccatcc cctgaaacct tagcaacccg 3540

gcatcagtaa gccccggcta tccattctaa cactgactct ggagaggagc tgctcacgct 3600

gcgcctccag caagccacag aaatctcctg gctcctccac ctcagcctct tcacctaaag 3660

tatctttctc tggggacaga tcccacccat cctcccgaca ctgctcccag gatcccccct 3720

gaactggctc tgtccttggc attggagctt atacatgccg attagtctgt ctctccagta 3780

aagccacaaa acccttaaag tcaaagcctg cccgtgtcct cctctagtgc ctcctcggta 3840

ccttagggca acgccactgt gggggaaacg ggagagaact tgaaccacag gctcggcgga 3900

ggaggccacc ctggggtaca acccctggtc ctctcacctg gatggctata ggcaagagtt 3960

gcccatcggg ctgcagcttc agcatgacga ggggggcagc caggtactgc tgactacaaa 4020

ggatgacatt ggccttgatc ccatccagaa ggaagaaatc cgcttcaaac agagtgcctt 4080

tctgcatagg gagagaaatc aagggcttct gtcagcatgg tcttctccca ccctgagtgc 4140

ccgacaagga gacaagaaag aagagagacg acctgtctcc atcagccacc tggcagtgtt 4200

tgtcctctac ctcatcaaag tctcctcatt cttcttcctt tttttcttct tactttttta 4260

ttgcatttgt ttgtttgctg ggtgggtgtg tatgttcgtg tatatgcata tatgtggaca 4320

catgtttgcc atgacatcgg gtgatgatca gaagacaacg tatgggaagt agctccctcc 4380

ttttaccaca caggtcccag ggatcaacta agattgttat gcttggcagc aggtagctct 4440

acctggcgtt accacaccta accttatatg ctccgtctaa attactccag attacaggaa 4500

aggaaaccga aagggtttcc cagaatcagg gtatcttggc aatcgggagc caaggaatac 4560

cccaaagtca caccatgcaa caaacctcac acatgagatt tattggggtg acagaacggc 4620

ggtggcctct gaacaggaac agagaaagca gcgagctgag tggaggggcg ggcttttaca 4680

gggtctttgg agcatgtgca gccagcgagt ggagcatgcg cagtgagcta gtgaaacagt 4740

acagagagcc tagaagtatt gcttgactgt gaaccttgaa taataggggt ggagatttcc 4800

aagtcccgag ttcaggacag gaccaggggc agggtgattt tccactggtc tattacccta 4860

aagaaacaga tcagagaaat gagatagtgc acttaaaatc acccagcaaa accaggattt 4920

agatgcaagt ctatctggcc ccatgaatct tgtcattgtc ccagcttggt tcctaattag 4980

ccccagccct gccttggatt ctctcaccct gtacctctct ggacccatgg cctccaggac 5040

aacgtcacat ctctactggc caccccaata aggctcctct cttcaaccat atactcctct 5100

cctgtgctgg gaaagccacg gtactacatt tttctacatc agtctaggat ttgtctggat 5160

cctctcttga caaccagcag gaattcacag taaatctgag cacagtacac ccaacaccca 5220

gtacacccaa cacccagtac acccaacacc cagtacaccc aacacccagt acacccaaca 5280

cccagtacac ccaacaccca gtacacccaa cacccagtac acccagtaca cccaacaccg 5340

gctcctgcct ccacttccct gcttggcctt cctggtgctt tgatgcccat accttgagct 5400

cctcatccag ctgggcctgt agcttctcca tccccggagg gaataccagg cgggcaggga 5460

gacaagtaga ccgcttcagc accatggggt tagcaccatt gagaaactgg tacccaaaga 5520

aagcatcctc tttccaggag tttcgaaccc gctctaggga taggaggagg acgtgtctaa 5580

gatccaactt cctggtcctt ccaacaccca ccaaaacatc cctacttgac ccagactacc 5640

agagatgtca gacaaaagcc tctgacccac ccaccctcaa cccggctttc tcgatctcct 5700

tgcattggga agagttttgg ggtaagagta atttgggggt aggtgtactt accagccatc 5760

ttggtgtggc cactcttgaa aacatagttg aagtcatcta ggcttttcca gcaggtcaca 5820

gtgtttttag ggaagttaat aacggtgtcc attgtccttc agggaagaat ggggcaaagg 5880

atgtgacagt tatacttaga accaaacatt acagccaaag gtaccataga cgagaccagc 5940

acacacccac cctcaagccg ggcactcttg ccctcacccc agaacctgtg aagcttcaaa 6000

ttcaagtctt ttgtcctctc gaaatcgctg gtctacaggg aggtcagaga tactggtcgc 6060

cgccacgttc aggattgtgc catccttcca gttgccccac ctgagggcag aagtgacatc 6120

aggcacgggc actgcagagg cagctcgcca tgggtcccct gggtcagcct cacctgtaca 6180

gactcctcct ttcttccagt tcctcctccc tgtggttcct gaacaggcct tgagagtctt 6240

caaccacggt gcagcctaga agccgaggag ggagacactc tggatgtctt ctgtgagctt 6300

ctctaccccc ttccagctgg ctgtacctct tcaggcagcc ctggctgtcc tctgtgcatg 6360

catcttcatg tccctccctg aagcctccgt gacatctggg ccagcttcag ctactctttc 6420

tttgacaact catttaaaac accaaacaaa cagcccagct tccaggctcc agctcccaca 6480

cgctgcctcc ccttcctcac acaactgagg ctccgttccc cctatgcctc ttcacacaca 6540

cacaccccac ccctctgctc accagtgccc tcagggaggt tcaggatgct ggtgccctga 6600

acccatcggt aacaggggaa cgtgtactcc gatccctggt ctccggggcc cttcacagaa 6660

atccagttgc agaaccaggc gtcctctttg agataatgcc atttctgcac tctcacaaac 6720

agcagtggcc caaggtattc tgacacatcc accttgaatt ctgcctcctg cagataagca 6780

caggggcgca gaatggggtg ctcaggctga gttctgcttc cactgggcca caggaaaagt 6840

gatgaaaaga ggctccatga caccactcag ggttgtcacc tcaaccaaga gtgagagggt 6900

taaggatgga cgatggacga tgggaaagta cactccttcc tccccggggg gtggggggag 6960

gactagagga aggggaattc tggaaactct gccagaggcc cagtgaggaa cgggtcaaat 7020

gggagacccc actaaggcag agaacccatg ggaattccat ttggcctgag ccagggtttc 7080

tacaggatgg tctccaggga gaaacctgga tttggagagg cggggttggt gagagagaat 7140

gaaatcagag aagacacgag gcagagtcca tggagagcgt agaggctgaa aggaccagcg 7200

ccttcatgaa gctagggccc ccagcgcctt catgaagcta gggcccagag agccgtgggg 7260

actgggagcc aaacaggacc aggtcccgag ggaccgagag taagccagag gcttggcagt 7320

tcgtagggaa gaagctggag acactgggag gctttatcag gcagactgtt ctacagtagg 7380

aacgagagaa gatggtaaat cttaagcttt aaaaaattag agacattcgg gcagctgaca 7440

ctgctgatac tggcactact actgtaccgc agagttctgt gaagtgaggg acgcacagaa 7500

atatcccact caatgaggcc aggaggcagc aactgttact gagcgctcac cgaatgcctc 7560

tgtgcaccct ttccgctttc cgaggctgtg agctccttcc tcagagttca ggggccacgc 7620

atcagggttc ctagggattt ctttgacttt gcccccgccc ccgcccccag cccctcaccc 7680

tgccgcctcc cgggacagcc agagctctgc gcagctcacc gagttccgac agggtcggaa 7740

cagcttcccg agagatgcct ccccatgctg tccgatcaac cacaggtaga cctcgttgtt 7800

ggagcccgcg tacacggagt ccccggtgga gacgcggatg cggtagacac ccatcttgca 7860

gatg 7864

<210> 2

<211> 1557

<212> DNA

<213> Mus musculus

<220>

<221> Mus musculus leukocyte-type 12-lipoxygenase gene

<222> (1)..(1557)

<223> GenBank accession no. U04332 as of 07 Jan 2003

<220>

<221> SNP

<222> (541)..(541)

<223>

<400> 2

gtgaaggtaa aaatcacaag cttggtacct cctgttgaaa tagttgtgat gcccattagc 60

aagcaggaca ggataggaac gtttaagaag aatagggcac atttgtgtca ccatggcatt 120

tctgtttgat tcgggagcag agagccttgc atttacagaa acagaacaat tcaggttagc 180

gaacgttcca gtaagtacca tacgtgcctg agagtaatgc tgacctcagt tccaccggcg 240

gtcgggtcag ggcttagcaa atatgtgatt cttatttcaa tggaaggggt gtgttagctt 300

ggggatgtgc acagaactga ctcatagtgg agattgccat tcaccagaca cattttcatt 360

gtctttgttt aattcagttt ataacactcc atgccagaat ggcaccaaag gtcattttat 420

tactcttatt agtcacatgg actgagagtc tttgtggtcc catgtctccc tagaggaagg 480

atgtatgtac agttgcattc agctcagtgg gttccaggtg tagcaacagt taacaataaa 540

cgaataagcc ctgctggatg cggtcttggc ctaggaggcg ctcctgtgag ccggtgatac 600

tgacacaagg atgatcaggg tccagcatta gccaggcatc agcagaagcg cttctggggt 660

aggcggtaga agctggttgt ctctgggtgg ggtgtagaag gcacttcact gagaagctag 720

agtctcctca ggatttggaa ggatgagtag ggtggacccc gagtacatga ttcttcagca 780

aataacatgg agtctttggt ggtggctctg gggagcaagg ggcttcagca ggagtgcagt 840

gtggggaagg gagaaaatca ggtttccgag ggctttgaag gccaggtaat ttcgggtttt 900

attatgcaga catcgggaga tatctggatg ctttgaaact cacagggatg agcccagagc 960

ctgcctcagg atgggcaaag tgtacagagc tggtagaatt aatcgggtgg ctaaaatgtg 1020

ccgcctttaa atgctgcaca gcacctgcar ccttaagatt atccctgggc atgggaaaac 1080

ccatctgtga tgcgcgcact ggagagtgga tgtatgtgcg tgtgagagtg tgtgtgtgtg 1140

tgtgtgtgtg tgtgtgtgtg tgtgagagag agagagagag agagagagag agagagagag 1200

agagagagag agagtgagcg cgcgtggagg aaggtgtgac gggggcagaa gggactggac 1260

cagatgttga ttctttcttg catgttttct tttcaaatcc ccatctactt ctccctcctg 1320

ttctcctctc accaagatga tttttgagag ttcagctttg cccagagccc cgcctttttt 1380

gggctggaac gaggccccca gcaagtgctg ggggccccag taagtgacag aggcggggtt 1440

aattgccacc tacagctcaa ccctaaacta gacttctgaa ggggcggggc ctcggctttc 1500

ttatttagcc cgtctacccc tcttgattct cagaagggag aaaacttcat ctgcaag 1557

## Description

The invention relates generally to 15-lipoxygenase inhibitors and their use in treating and preventing bone loss. Specifically, the invention relates to the use of 15-lipoxygenase inhibitors to decrease bone loss and/or increase net bone formation.

Lipoxygenases are nonheme iron-containing enzymes found in plants and animals that catalyze the oxygenation of certain polyunsaturated fatty acids, such as lipids and lipoproteins. Several different lipoxygenase enzymes are known, each having a characteristic oxidation action. Mammalian lipoxygenases are named by the position in arachidonic acid that is oxygenated. The enzyme 5-lipoxygenase converts arachidonic acid to 5-hydroperoxyeicosatetraenoic acid (5-HPETE). This is the first step in the metabolic pathway which yields 5-hydroxyeicosatetraenoic acid (5-HETE) and the leukotrienes (LTs). Similarly, 12- and 15-lipoxygenase convert arachidonic acid to 12- and 15-HPETE, respectively. Biochemical reduction of 12-HPETE leads to 12-HETE, while 15-HETE is the precursor of the class of compounds known as lipoxins.

A diverse array of biological effects are associated with the products of lipoxygenase activity, and many are implicated as mediators in various disease states. The C4 and D4 LTs are potent constrictors of human bronchial smooth muscle; LTB4 and 5-HETE, found in the synovial fluid of patients with rheumatoid arthritis, are potent chemotactic factors for inflammatory cells such as polymorphonuclear leukocytes (Green and Lambeth, Tetrahedron, 39, 1687 (1983)); 12-HETE has been found at high levels in the epidermal tissue of patients with psoriasis; the lipoxins have been shown to stimulate lysosomal enzyme and superoxide ion release from neutrophils. Thus, lipoxygenase enzymes play an important role in the biosynthesis of mediators of asthma, allergy, arthritis, psoriasis, and inflammation, and inhibitors of these enzymes interrupt the biochemical pathway involved in these disease states.

Human 15-lipoxygenase (15-LO) catalyzes the formation of 15-S-hydroxyeicosatetraenoic acid (15-S-HETE) from arachidonic acid (Kuhn and Borngraber, *Lipoxygenases and Their Metabolites,* Plenum Press, New York, (1999)). In mice, the synthesis of 15-S-HETE is carried out by 12/15-Lipoxygenase (Alox15), which is the murine homologue of human 15-LO. Murine 12/15-LO converts arachidonic acid to 12(S)-hydroxyeicosatetraenoic and 15-S-HETE in a 3:1 ratio, and can additionally convert linoleic acid to 13-hydroxyoctadecadienoic acid (13-HODE).

15-Lipoxygenase has previously been implicated in the pathogenesis of several diseases, including atherosclerosis (Harats et al., *Arterioscler. Thromb. Vasc. Biol.,* 2100-2105, (2000)), asthma (Shannon et al., *Am.Rev.Respir. Dis.,* 147, 1024-1028, (1993)), cancer (Shureiqi et al., *JNCI*, 92, 1136-1142, (2000)), and glomerulonephritis (Montero and Badr, *Exp. Neph.,* 8, 14-19 (2000)). A number of classes of compounds have been identified that inhibit 15-lipoxygenase, including phenols, hydroxamic acids and acetylenic fatty acids (reviewed in Kuhn and Borngraber, *Lipoxygenases and Their Metabolites,* Plenum Press, New York, (1999)). The spectrum of inhibitory activities varies for these agents. For example, nordihydroguaiaretic acid has been shown to be an inhibitor of 5- and 15-lipoxygenase, naphthylhydroxamic acids have been shown to inhibit 5-, 12-, and 15-lipoxygenase (U.S. Patent No. 4,605,669), and a benzofluorene 15-lipoxygenase inhibitor, PD146176, has been reported to be relatively specific for the 15-lipoxygenase enzyme (Sendobry et al., *Br. J. Pharm.,* 120, 1199-1206, (1997)). Evidence for 15-lipoxygenase involvement in atherosclerosis has come from studies with mice with a targeted deletion of Alox15 (Alox15 -/-). These Alox15 knockout mice were initially shown to have minor phenotypic differences including increased 5-LO activity (Sun and Funk, *J. Biol. Chem*., 271, 24055-24062, (1996)). However, disruption of Alox15 greatly diminished atherosceloritic lesions in apoE deficient (apoE -/-) atherosclerosis prone mice (Cyrus et al., *J. Clin Invest.,* 103, 1597-1604, (1999)).

Although 5-LO has been implicated in the pathogenesis of several diseases, the biologic function of murine or human 15-lipoxygenase has not been determined with certainty, nor has a human clinical utility for inhibitors of 15-lipoxygenase been established. In particular, the utility of 15-lipoxygenase inhibitors for treatment of human osteoporosis and/or osteoarthritis has not previously been discovered.

Osteoporosis is caused by a reduction in bone mineral density in mature bone and results in fractures after minimal trauma. The disease is widespread and has a tremendous economic impact. The most common fractures occur in the vertebrae, distal radius and hip. An estimated one-third of the female population over age 65 will have vertebral fractures, caused in part by osteoporosis. Moreover, hip fractures are likely to occur in about one in every three woman and one in every six men by extreme old age.

Two distinct phases of bone loss have been identified. One is a slow, age-related process that occurs in both genders and begins at about age 35. This phase has a similar rate in both genders and results in losses of similar amounts of cortical and cancellous (spongy or lattice like) bone. Cortical bone predominates in the appendicular skeleton while cancellous bone is concentrated in the axial skeleton, particularly the vertebrae, as well as in the ends of long bones. Osteoporosis caused by age-related bone loss is known as Type II osteoporosis.

The other type of bone loss is accelerated, seen in postmenopausal women and is caused by estrogen deficiency. This phase results in a disproportionate loss of cancellous bone. Osteoporosis due to estrogen depletion is known as Type I osteoporosis. The main clinical manifestations of Type I osteoporosis are vertebral, hip and forearm fractures. The skeletal sites of these manifestations contain large amounts of trabecular bone. Bone turnover is usually high in Type I osteoporosis. Bone resorption is increased but there is inadequate compensatory bone formation. Osteoporosis has also been related to corticosteroid use, immobilization or extended bed rest, alcoholism, diabetes, gonadotoxic chemotherapy, hyperprolactinemia, anorexia nervosa, primary and secondary amenorrhea, transplant immunosuppression, and oophorectomy.

The mechanism by which bone is lost in osteoporosis is believed to involve an imbalance in the process by which the skeleton renews itself. This process has been termed bone remodeling. It occurs in a series of discrete pockets of activity. These pockets appear spontaneously within the bone matrix on a given bone surface as a site of bone resorption. Osteoclasts (bone dissolving or resorbing cells) are responsible for the resorption of a portion of bone of generally constant dimension. This resorption process is followed by the appearance of osteoblasts (bone forming cells) which then refill the cavity left by the osteoclasts with new bone.

In a healthy adult subject, osteoclasts and osteoblasts function so that bone formation and bone resorption are in balance. However, in osteoporosis an imbalance in the bone remodeling process develops which results in bone being replaced at a slower rate than it is being lost. Although this imbalance occurs to some extent in most individuals as they age, it is much more severe and occurs at a younger age in postmenopausal osteoporosis, following oophorectomy, or in iatrogenic situations such as those resulting from the use of corticosteroids or immunosuppressants.

Various approaches have been suggested for increasing bone mass in humans afflicted with osteoporosis, including administration of androgens, fluoride salts, and parathyroid hormone and modified versions of parathyroid hormone. It has also been suggested that bisphosphonates, calcitonin, calcium, 1,25-dihydroxy vitamin D₃, and/or estrogens, alone or in combination, may be useful for preserving existing bone mass.

The present invention is based on the discovery that inhibitors of 15-lipoxygenase are able to increase net bone formation and/or enhance bone accretion and enhance fracture healing. These molecules can be delivered alone or in combination with additional agents which inhibit bone resorption and/or enhance bone formation, particularly anabolic agents

Accordingly, in one embodiment, the subject invention is directed to a method for reducing bone loss in a subject. The method comprises administering to the subject a pharmaceutically effective amount of an inhibitor of 15-lipoxygenase.

In another embodiment, the invention is directed to a method for increasing bone mineral density, which comprises administering to the subject an effective amount of a pharmaceutically acceptable inhibitor of 15-lipoxygenase.

In another embodiment, the invention is directed to a method of diagnosis of predisposition to bone loss in a subject, the method comprising detecting a polymorphism on human chromosome 17, particularly detecting a polymorphism in the 15-lipoxygenase gene.

In yet another embodiment, the invention is directed to a method of screening for compounds for reducing bone loss or for increasing bone mineral density by contacting inhibitors of 15-lipoxygenase with human mesenchymal stem cells and determining cellular differentiation into cells involved in bone formation.

These and other aspects of the present invention will become evident upon reference to the following detailed description and attached figures. In addition, various references are set forth herein which describe in more detail certain procedures or compositions, and are therefore incorporated by reference in their entirety.

The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, *Proteins: Structures and Molecular Properties* (W.H. Freeman and Company, 1993); A.L. Lehninger, *Biochemistry* (Worth Publishers, Inc., current addition); Sambrook, et al., *Molecular Cloning: A Laboratory Manual* (2nd Edition, 1989); *Methods In Enzymology* (S. Colowick and N. Kaplan eds., Academic Press, Inc.); *Remington's Pharmaceutical Sciences,* 18th Edition (Easton, Pennsylvania: Mack Publishing Company, 1990); Carey and Sundberg *Advanced Organic Chemistry 3*^{*rd*} *Ed.* (Plenum Press) Vols A and B(1992).

All publications, patents and patent applications cited herein, whether *supra* or *infra*, are hereby incorporated by reference in their entirety.

In describing the present invention, the following terms will be employed, and are intended to be defined as indicated below.

By "bone loss" is meant an imbalance in the ratio of bone formation to bone resorption resulting in less bone than desirable in a patient. Bone loss may result from osteoporosis, osteotomy, periodontitis, or prosthetic loosening. Bone loss may also result from secondary osteoporosis which includes glucocorticoid-induced osteoporosis, hyperthyroidism-induced osteoporosis, immobilization-induced osteoporosis, heparin-induced osteoporosis or immunosuppressive-induced osteoporosis. Bone loss can be monitored, for example, using bone mineral density measurements described below.

The terms "effective amount" or "pharmaceutically effective amount" refer to a nontoxic but sufficient amount of the agent to provide the desired biological result. That result can be reduction and/or alleviation of the signs, symptoms, or causes of a disease, or any other desired alteration of a biological system. For example, an "effective amount" for therapeutic uses is the amount of the composition comprising an active compound herein required to provide a clinically significant increase in healing rates in fracture repair; reversal of bone loss in osteoporosis; reversal of cartilage defects or disorders; prevention or delay of onset of osteoporosis; stimulation and/or augmentation of bone formation in fracture non-unions and distraction osteogenesis; increase and/or acceleration of bone growth into prosthetic devices; and repair of dental defects. An appropriate "effective" amount in any individual case may be determined by one of ordinary skill in the art using routine experimentation.

By "increased bone accretion" is meant that bone accumulation in a subject administered the 15-lipoxygenase inhibitors of the invention is increased over bone accumulation in a comparable subject that is not given an 15-lipoxygenase inhibitor. Such increased bone accretion is typically determined herein by measuring bone mineral density (BMD). For example, bone accretion can be determined using an animal model, such as an ovariectomized mouse, dog and the like. The animal is administered the test compound and bone mineral density (BMD) measured in bones that are normally depleted in Type I or Type II osteoporosis, such as bones of the appendicular and/or axial skeleton, particularly the spine including the vertebrae, as well as in the ends of long bones, such as the femur, midradius and distal radius. Several methods for determining BMD are known in the art. For example, BMD measurements may be done usingdual energy x-ray absorptiometry or quantitative computed tomography, and the like. (See, the examples.) Similarly, increased bone formation can be determined using methods well known in the art. For example, dynamic measurements of bone formation rate (BFR) can be performed on tetracycline labeled cancellous bone from the lumbar spine and distal femur metaphysis using quantitative digitized morphometry (see, e.g., Ling et al., *Endocrinology* ( 1999) 140:5780-5788. Alternatively, bone formation markers, such as alkaline phosphatase activity and serum osteocalcin levels can be assessed to indirectly determine whether increased bone formation has occurred (see Looker *et al*., *Osteoporosis International* (2000) 11(6):467-480).

By "increased bone formation" is meant that the amount of bone formation in a subject administered the 15-lipoxygenase inhibitors of the invention is increased over the bone formation rate in a subject that is not given an 15-lipoxygenase inhibitor. Such enhanced bone formation is determined herein using, e.g., quantitative digitized morphometry, as well as by other markers of bone formation, as described above.

By "inhibitor of 15-lipoxygenase" is meant a compound that inhibits 15-lipoxygenase with an IC50 of less than 1µM, preferably less than 100nM. IC50's may be determined by standard methods. One particular method is a colorimetric assay in which the putative inhibitor is pre-incubated with the 15-lipoxygenase enzyme for about 10 minutes followed by addition of linoleic acid substrate for an additional 10 minutes. The product, 13-HPODE, is quantitated by coupling the reduction of the hydroperoxylated lipid to the oxidation on N-benzoyl-leucomethylene blue in the presence of hemin at pH 5. The absorbance of the oxidized methylene blue is directly proportional to the amount of 13-HPODE formed by the 15-lipoxygenase, and can be measured in the presence and absence of inhibitor. This is an endpoint assay described in more detail in "A Spectrophotometric Microtiter-Based Assay for the Detection of Hydroperoxy Derivatives of Linoleic Acid", Analytical Biochemistry,201, 375-380 (1992); Bruce J. Auerbach, John S. Kiely and Joseph A. Cornicelli. Other assays include those where the initial enzyme reaction rate is measured spectrophotometrically by measuring conjugated diene formation at 234nm.

As used herein, the terms "treat" or "treatment" are used interchangeably and are meant to indicate a postponement of development of bone loss symptoms and/or a reduction in the severity of such symptoms that will or are expected to develop. The terms further include preventing additional symptoms, ameliorating or preventing the underlying metabolic causes of symptoms, and/or encouraging bone growth.

By "pharmaceutically acceptable" or "pharmacologically acceptable" is meant a material which is not biologically or otherwise undesirable, i.e., the material may be administered to an individual without causing any undesirable biological effects or interacting in a deleterious manner with any of the components of the composition in which it is contained.

By "physiological pH" or a "pH in the physiological range" is meant a pH in the range of approximately 7.2 to 8.0 inclusive, more typically in the range of approximately 7.2 to 7.6 inclusive.

As used herein, the term "subject" encompasses mammals and non-mammals. Examples of mammals include, but are not limited to, any member of the Mammalia class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. The term does not denote a particular age or gender.

As used herein, "polymorphism" refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements.

A single nucleotide polymorphism (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1 000 members of the populations). A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

As used herein, the term "precursor cell" refers to a cell that is not fully differentiated or committed to a differentiation pathway, and that generally does not express markers or function as a mature, fully differentiated cell.

As used herein, the term "mesenchymal cells" or "mesenchymal stem cells" refers to pluripotent progenitor cells that are capable of dividing many times, and whose progeny will give rise to skeletal tissues, including cartilage, bone, tendon, ligament, marrow stroma and connective tissue (see A. Caplan J. Orthop. Res. (1991) 9:641-50).

As used herein, the term "osteogenic cells" includes osteoblasts and osteoblast precursor cells.

As used herein, "Quantitative Trait Locus" (QTL) refers to a phenotypic measure, such as bone mineral density, that is continuously distributed and can be determined by multiple genes. A QTL is a site on a chromosome whose alleles influence a quantitative trait.

The compounds used in the present invention are those that inhibit or reduce the activity of 15-lipoxygenase. In this context, inhibition and reduction of the enzyme activity refers to a lower level of measured activity relative to a control experiment in which the enzyme, cell, or subject is not treated with the test compound. In particular embodiments, the inhibition or reduction in the measured activity is at least a 10% reduction or inhibition. One of skill in the art will appreciate that reduction or inhibition of the measured activity of at least 20%, 50%, 75%, 90% or 100% or any integer between 10% and 100%, may be preferred for particular applications. Typically, the 15-lipoxygenase inhibitors used in this invention will have IC50's of less than 1µM, preferably less than 100nM.

The present invention is based, in part, on the discovery that 15-lipoxygenase is an important modulator of bone mass. In particular, a genome scan of 100 microsatellite markers in mice led to the identification of bone loss disorder susceptibility loci on chromosomes 1, 2, 4, and 11. The differences in gene expression, especially for genes encoded on chromosome 11, identified substantial differences in expression of a gene encoding for 15-lipoxygenase, thereby linking the expression of 15-lipoxygenase to a lowered bone mineral density.

Peak bone mass is a major determinant of osteoporotic fracture risk. Family and twin studies have shown that genetic factors regulate bone mineral density (BMD) (reviewed in Stewart and Ralston, *J. Endocr.,* (2000) 166: 235-245). The inventors herein utilized a murine genetic model to identify loci that control BMD. A two step method was used to identify the regions of the genome that regulate bone mineral density. Initially, a computational method and microsatellite markers were used to scan an animal single nucleotide polymorphism (SNP) database, and the chromosomal regions that contributed to acquisition and maintenance of skeletal mass were predicted (Example 1). Subsequently, animal models were used to identify and validate the mutations in the genes contributing to the acquisition and maintenance of skeletal mass (Example 2) and to show that disruption of the 15-lipoxygenase gene causes increased BMD (Example 4), thereby identifying 15-lipoxygenase as a gene involved in the regulation of bone mass.

Compounds that inhibit 15-lipoxygenase activity and prevent bone resorption or promote bone formation provide important benefits to efforts at treating osteoporosis. Compounds that inhibit 15-lipoxygenase activity can be used in a method for treating osteoporosis or osteoarthritis by inhibition of osteoclastic bone resorption or by stimulation of osteoblast differentiation and promotion of new bone formation. Example 3 shows the ability of 15-lipoxygenase inhibitors promote the differentiation of human mesenchymal stem cells into osteoblasts *in vitro.* Example 4 shows that total disruption of the 15-lipoxygenase gene *in vivo* leads to increased BMD. Example 5 shows the ability of 15-lipoxygenase inhibitors to promote bone formation in an *in vivo* model.

According to the present invention, an inhibitor of 15-lipoxygenase can be used for the manufacture of a medicament for reducing bone loss in a mammalian subject, comprising an effective amount of said inhibitor of 15-lipoxygenase. Thus, the present invention provides for a method for reducing bone loss in a mammalian subject, the method comprising administering an effective amount of an inhibitor of 15-lipoxygenase to the subject. Preferably, bone loss is associated with osteoporosis, osteoarthritis, Paget's disease, or periodontal diseases. More perferably, bone loss is associated with osteoporosis.

Further to this, according to the present invention, an inhibitor of 15-lipoxygenase can be used for the manufacture of a medicament for increasing bone mineral density in a mammal comprising an effective amount of said inhibitor of 15-lipoxygenase. Thus, the present invention also provides a method of increasing bone mineral density in a mammal by administering to the mammal an effective amount of an inhibitor of 15-lipoxygenase.

According to the present invention, an inhibitor of 15-lipoxygenase can be used for the manufacture of a medicament for the treatent of osteoporosis in a mammal comprising an effective amount of said inhibitor of 15-lipoxygenase. Thus, the present invention provides a method of treating osteoporosis in a mammal by administering to the mammal an effective amount of an inhibitor of 15-lipoxygenase.

Furthermore, an inhibitor of 15-lipoxygenase can be used for the manufacture of a medicament for promoting differentiation of mesenchymal stem cells in a human to osteoblasts comprising an effective amount of said inhibitor of 15-lipoxygenase to increase the osteoblasts in said human. Thus, the present invention provides a method of promoting differentiation of mesenchymal stem cells in a human to osteoblasts comprising administering to the human an effective amount of an inhibitor of 15-lipoxygenase to increase the osteoblasts in said human. Preferably, the inhibitor of 15-lipoxygenase has an IC50 of less than 1 µM.

Treatment with a 15-lipoxygenase inhibitor may be used for healing of bone fractures and osteotomies, including both union and nonunion fractures. Types of fractures treatable by the methods of this invention include both traumatic and osteoporotic fractures, e.g., fractures of the hip, neck of the femur, wrist, vertebrae, spine, ribs, sternum, larynx and trachea, radius/ulna, tibia, patella, clavicle, pelvis, humerus, lower leg, fingers and toes, face and ankle. Both the rate of healing as well as promotion of union in a fracture that would otherwise remain as a nonunion fracture may be facilitated by the methods disclosed herein. Prophylactic treatment of a patient identified to be at risk for fracture may also reduce the fracture risk of that patient.

Accordingly, an inhibitor of 15-lipoxygenase may be used for the manufacture of a medicament for reducing the incidence of fracture in a mammal comprising an effective amount of said inhibitor of 15-lipoxygenase. Thus, the present invention provides a method of reducing the incidence of fracture in a mammal by administering to the mammal an effective amount of an inhibitor of 15-lipoxygenase.

Furthermore, an inhibitor of 15-lipoxygenase can be used for the manufacture of a medicament for healing fractures in a mammal comprising an effective amount of said inhibitor of 15-lipoxygenase. Thus, the persent invention also provides a method of healing fractures in a mammal by administering to the mammal an effective amount of an inhibitor of 15-lipoxygenase.

Preferably, the mammal of the methods and uses hereinbefore described is a human. More preferably, the inhibitor of 15-lipoxygenase hereinbefore described has an IC50 of less than 1 µM.

Several inhibitors of 15-lipoxygenase are known which may find use with the subject methods. The inhibitors include synthetic organic molecules, plant extracts and other natural products, and antibodies against 15-lipoxygenase. Representative and nonlimiting examples are described in Cornicelli JA, Trivedi BK. 15-lipoxygenase and its inhibition: a novel therapeutic target for vascular disease. [Review] [113 refs]. *Current Pharmaceutical Design* 1999; 5(1):11-20 (describing various caffeic acid derivatives, propargyl ethers, catechols and benzothiopyranoindoles); Cornicelli JA. 15-lipoxygenase inhibitors as antiatherosclerotic agents. *IDrugs* 1998; 1(2):206-213; Fleischer R, Frohberg P, Buge A, Nuhn P, Wiese M. QSAR analysis of substituted 2-phenylhydrazonoacetamides acting as inhibitors of 15-lipoxygenase. *Quant Struct -Act Relat* 2000; 19(2):162-172; Kuhn H. Inhibitors of 12/15-lipoxygenase are potential anti-atherosclerotic drugs. *Curr Opin Anti-Inflammatory Immunomodulatory Invest Drugs* 1999; 1(3):227-237; Mogul R, Johansen E, Holman TR. Oleyl sulfate reveals allosteric inhibition of soybean lipoxygenase-1 and human 15-lipoxygenase. *Biochemistry* 2000; 39(16):4801-4807; Sexton K, Roark WH, Sorenson R, Cornicelli J, Sekerke C, Welch K. Thiourea inhibitors of 15-lipoxygenase. *Abstracts of Papers American Chemical Society* 1999; 218(1-2):MEDI0; Tait BD, Dyer RD, Auerbach BJ, Bornemeier D, Guilds-Zamarka L, Oxender M et al. Catechol based inhibitors of 15-lipoxygenase. *Bioorganic & Medicinal Chemistry Letters* Vol 1996; 6( 1 ):93-96; Moreau RA, Agnew J, Hicks KB, Powell MJ. Modulation of lipoxygenase activity by bacterial hopanoids. *Journal of Natural Products* Vol 1997; 60(4):397-398; *219th National Meeting of the American Chemical Society (2000), Poster BIOL-15.* Authors: E-N-Jonsson and T-R-Holman. University of California, Santa Cruz, CA (describing 15-lipoxygenase inhibitors isolated from marine sponges; and Lyckander IM, Malterud KE. Lipophilic flavonoids from Orthosiphon spicatus as inhibitors of 15-lipoxygenase. *Acta Pharm Nord* 1992; 4(3):159-166. In addition, the thiourea and benzamide compounds of U.S. Patent No. 6,268,387, to Conner *et al*. (such as represented by3-amino-N-(3,4-dichlorophenyl)-4-methoxy-benzamide Compound 3) which are inhibitors of 15-lipoxygenase, as well as 2-phenyl-benzo [d] isoselenazol-3-one (ebselen), 6,11-dihydro-5-thia-11-aza-benzo[a]fluorine (termed Compound 2 herein), and phenyl acetylenic compounds represented by, 3-(2-oct-1-ynyl-phenyl)-acrylic acid Compound 4 are also useful in the methods described herein. Also useful are compounds disclosed in WO01/96298 (incorporated by reference), including Compound 6, [ [ [5-(5,6-difluoro-1H-indol-2-yl)-2-methoxyphenyl]amino]sulfonyl]-carbamic acid-isobutyl ester.

Additional 15-lipoxygenase inhibitors include those shown below;
1) 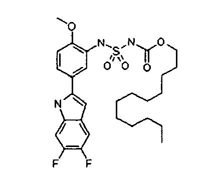 described in 222nd National Meeting of the American Chemical Society, Chicago, Illinois, USA, 26 - 30 August, 2001. Poster, MEDI 270.
2) PD 148104 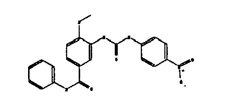 described in 218th ACS (New Orleans), 1999, MEDI 200.
3) PD 146176 from Parke Davis (now Pfizer)
4) A 78773 (Abbott) described in WO 92/1682
5) QA-208-199 (Novartis) 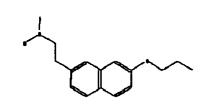
described at 6th Int Conf Prostaglandins (Florence), 1986, 328

A related aspect of this invention relates to combination therapies of 15-lipoxygenase inhibitors for increased bone formation with other active agents such as bisphosphonates, estrogen, SERMS (selective estrogen receptor modulators), calcitonins or anabolic therapies. Examples of bisphosphonates include alendronate, ibandronate, pamidronate, etidronate and risedronate. Examples of SERMS include raloxifene, dihydroraloxifene and lasofoxifene. Calcitonins include human and salmon calcitonin. Anabolic agents include parathyroid hormones (PTH) e.g. hPTH(1-34), PTH(1-84), and parathyroid hormone-related protein (PTHrP) and analogs thereof. Particular analogs of PTHrP are described in "Mono- and Bicyclic Analogs of Parathyroid Hormone-Related Protein. 1. Synthesis and Biological Studies," Michael Chorev et al. Biochemistry, 36:3293-3299 (1997) and "Cyclic analogs of PTH and PTHrP," WO 96/40193 and U.S. Patent No. 5,589,452 and WO 97/07815. The other active agent may be administered concurrently, prior to or after the 15-lipoxygenase inhibitor and may be administered by a different delivery method. Preferably, the 15-lipoxygenase inhibitor is administered first. The period of this administration may be of any length, but typically ranges from six to twenty four months. This treatment is then followed by treatment with an antiresorptive agent, e.g., a bisphosphonate, SERM, calcitonin or hormone replacement therapy.

Thus, preferably, an inhibitor of 15-lipoxygenase can be used as hereinbefore described, comprising an additional active agent. Accordingly, the method hereinbefore described preferably comprises an additional active agent. More preferably, said active agent comprised in the use or method is effective to regulate calcium resorption from bone. Most preferably, said active agent is selected from the group consisting of an estrogen, a calcitonin, a bisphosphonate and an IGF.

In a preferred embodiment, the 15-lipoxygenase inhibitor of the uses and methods hereinbefore described is selected from the group consisting of synthetic organic molecules, natural products and antibodies against 15-lipoxygenase. In a more preferred embodiment, the 15-lipoxygenase inhibitor is selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester.

In one embodiment, a method of the present invention involves the administration of a therapeutically effective amount of an antisense oligonucleotide having a sequence capable of binding specifically with any sequences of genomic DNA or an mRNA molecule which encodes 15-lipoxygenase, so as to prevent transcription or translation of 15-lipoxygenase mRNA. By "antisense" is meant a composition containing a nucleic acid sequence which is complementary to the "sense" strand of a specific nucleic acid sequence. Once introduced into a cell, the complementary nucleotides combine with endogenous sequences produced by the cell to form duplexes and to block either transcription or translation. See, e.g., Agrawal, S., ed. (1996) Antisense Therapeutics, Humana Press Inc., Totawa NJ; Alama et al. (1997) Pharmacol. Res. 36:171-178; Crooke, S.T. (1997) Adv. Pharmacol. 40:1-49; and Lavrosky et al. (1997) Biochem. Mol. Med. 62(1):11-22. Antisense sequences can be any nucleic acid material, including DNA, RNA, or any nucleic acid mimics or analogs. See, e.g., Rossi et al. (1991) Antisense Res. Dev. 1:285-288; Pardridge et al. (1995) Proc. Nat. Acad. Sci. 92:5592-5596; Nielsen and Haaima (1997) Chem. Soc. Rev. 96:73-78; and Lee et al. (1998) Biochemistry 37:900-1010. Delivery of antisense sequences can be accomplished in a variety of ways, such as through intracellular delivery using a recombinant vector.

Antisense oligonucleotides of about 15 to 25 nucleic acid bases are typically preferred as such are easily synthesized and are capable of producing the desired inhibitory effect. Molecular analogs of antisense oligonucleotides may also be used for this purpose and can have added advantages such as stability, distribution, or limited toxicity advantageous in a pharmaceutical product. In addition, chemically reactive groups, such as iron-linked ethylenediamine-tetraacetic acid (EDTA-Fe), can be attached to antisense oligonucleotides, causing cleavage of the RNA at the site of hybridization. These and other uses of antisense methods to inhibit the *in vitro* translation of genes are well known in the art. See, e.g., Marcus-Sakura (1988) Anal. Biochem. 172:289.

While a number of 15-lipoxygenase inhibitors are described herein and known, it is understood that many others may be used in the subject methods. The methods described herein are intended to include the use of currently known 15-lipoxygenase inhibitors as well as those discovered subsequently. The assays described herein and those known to one of skill in the art enable other 15-lipoxygenase inhibitors that are useful for preventing or treating bone loss to be readily identified and developed.

As shown in the Examples, genetic abnormalities or alleles were found in chromosome 11, and the position of the allele corresponded to the gene for 15-lipoxygenase. Thus, inhibition or induction of 15-lipoxygenase has applications in various situations. It is possible that by inhibiting 15-lipoxygenase intracellularly, one may reduce bone turnover and thereby increase bone mineral density and bone quality. Thus, inhibition of 15-lipoxygenase may be used in a method for treating or preventing excessive resorption of bone, such as occurs in osteoporosis and osteoarthritis.

The 15-lipoxygenase inhibitors of the present invention may also be used to stimulate growth of bone-forming cells or their precursors, or to induce differentiation of bone-forming cell precursors, either *in vitro* or *ex vivo.* More particularly, 15-lipoxygenase inhibitors are useful for stimulating a cell population containing marrow mesenchymal cells, thereby increasing the number of osteogenic cells in that cell population. In a preferred method, hematopoietic cells are removed from the cell population, either before or after treatment with 15-lipoxygenase inhibitors. Through practice of such methods, osteogenic cells may be expanded. The expanded osteogenic cells can be infused (or reinfused) into a vertebrate subject in need thereof. For instance, a subject's own mesenchymal stem cells can be exposed to compounds of the present invention *ex vivo,* and the resultant osteogenic cells could be infused or directed to a desired site within the subject, where further proliferation and/or differentiation of the osteogenic cells can occur without immunorejection. Alternatively, the cell population exposed to the 15-lipoxygenase inhibitors may be immortalized human fetal osteoblastic or osteogenic cells. If such cells are infused or implanted in a vertebrate subject, it may be advantageous to immunoprotect these non-self cells, or to immunosuppress (preferably locally) the recipient to enhance transplantation and bone or cartilage repair.

The effectiveness of treatment can be followed by a method of monitoring the efficacy of the uses and methods hereinbefore described in a subject comprising measuring the level of 15-lipoxygenase in the subject, which is also provided by the present invention.

Several methods for identifying classes of 15-lipoxygenase inhibitors that may also prevent bone loss and/or promote bone formation may be employed. One method used to identify compounds that inhibit 15-lipoxygenase activity involves placing cells, tissues, or preferably a cellular extract or other preparation containing 15-lipoxygenase in contact with several known concentrations of a test compound in a buffer compatible with 15-lipoxygenase activity. The level of 15-lipoxygenase activity for each concentration of test compound is measured by quantitation of enzyme product and the IC₅₀ (the concentration of the test compound at which the observed activity for a sample preparation is observed to fall one-half of its original or a control value) for the compound is determined using standard techniques. Other methods for determining the inhibitory concentration of a compound of the invention against 15-lipoxygenase are known to one of skill in the art and can be employed as will be apparent based on the disclosure herein. Specific assays that may be used to identify 15-lipoxygenase inhibitors include those described in U.S. Patent No. 6,268,387B1 and 5,958,950 (rabbit reticulocyte assay) and U.S. Patent No. 4,623,661 (radiolabelled arachidonic acid in RBL-1 cells)

For example, antagonists can normally be found once a ligand has been structurally defined. Testing of potential ligand analogs is now possible upon the development of highly automated assay methods using physiologically responsive cells. In particular, new agonists and antagonists will be discovered by using screening techniques described herein.

In another method, rational drug design, based upon structural studies of the molecular shapes of the chemokines, other effectors or analogs, or the receptors, may be used to identifying compounds whose three-dimensional structure is complementary to that of the active site of 15-lipoxygenase. These compounds may be determined by a variety of techniques including molecular mechanics calculations, molecular dynamics calculations, constrained molecular dynamics calculations in which the constraints are determined by NMR spectroscopy, distance geometry in which the distance matrix is partially determined by NMR spectroscopy, x-ray diffraction, or neutron diffraction techniques. In the case of all these techniques, the structure can be determined in the presence or absence of any ligands known to interact with 15-lipoxygenase.

Such computer programs include but are not limited to AMBER (available from University of California, San Francisco), CHARMM (Chemistry at HARvard Molecular Mechanics, available from Harvard University), MM2, SYBYL (Trypos Inc.), CHEMX (Chemical Design), MACROMODEL, GRID (Molecular Discovery Ltd), and Insight II (Accelryl). Such programs are contemplated as being useful for the determination of the chemical interaction between two molecules, either isolated, or surrounded by solvent molecules, such as water molecules, or using calculations that approximate the effect of solvating the interacting molecules. The relative orientation of the two can be determined manually, by visual inspection, or by using other computer programs which generate a large number of possible orientations. Examples of computer programs include but are not limited to DOCK and AutoDOCK. Each orientation can be tested for its degree of complementarity using the computer programs. Thus, novel compounds can be designed that are capable of inhibiting 15-lipoxygenase.

Another method for identifying compounds that may inhibit 15-lipoxygenase involves the use of techniques such as UV/VIS spectroscopy, polarimetry, CD or ORD spectroscopy, IR or Raman spectroscopy, NMR spectroscopy, fluorescence spectroscopy, HPLC, gel electrophoresis, capillary gel electrophoresis, dialysis, refractometry, conductometry, atomic force microscopy, polarography, dielectometry, calorimetry, solubility, EPR or mass spectroscopy. The application of these methods can be direct, in which the compound's interaction with 15-lipoxygenase is measured directly, or it can be indirect, in which a particular agent having a useful spectroscopic property is used as a probe for the ability of other compounds to bind to 15-lipoxygenase; for example, by displacement or by fluorescence quenching.

Accordingly, the present invention provides a method for identifying compounds that increase bone mineral density, the method comprising contacting a compound with 15-lipoxygenase and determining whether the compound inhibits 15-lipoxygenase activity. Preferably, said method further comprises testing the compound in a functional assay that demonstrates an effect of the compound on bone formation. More preferably, the functional assay comprises contacting the compound with human mesenchymal stem cells and determining cellular differentiation into bone forming cells. In another more preferred embodiment, the functional assay comprises administering the compound to a non-human animal and measuring an index of bone formation. In a most preferred embodiment, the index measured is bone mineral density. In another most preferred embodiment, the index is a biomechanical parameter of bone.

In another most preferred embodiment, the , the functional assay comprises contacting the compound with human mesenchymal stem cells and determining cellular differentiation into bone forming cells, wherein determining cellular differentiation comprises performing an alkaline phosphatase assay, a calcium assay, a total DNA preparation assay, or combinations thereof.

In another embodiment, the present invention provides a method for identifying compounds that increase bone mineral density, the method comprising contacting an inhibitor of 15-lipoxygenase with human mesenchymal stem cells and determining cellular differentiation into bone forming cells. Preferably, determining cellular differentiation comprises performing an alkaline phosphatase assay, a calcium assay, a total DNA preparation assay, or combinations thereof.

The 15-lipoxygenase inhibitors thus identified or designed can be subsequently tested for their ability to prevent bone loss and/or promote bone formation. In one embodiment, the computer based methods discussed above are used. In another method, the compounds are tested for their ability to modulate known targets for bone loss, such as, for example, estrogen receptors, tumor necrosis factor receptor, integrin receptor, and the like. In yet another method, the compounds are tested for their ability to differentiate stem cells into bone cells.

The methods described herein use pharmaceutical compositions comprising the molecules described above, together with one or more pharmaceutically acceptable carriers, and optionally other therapeutic and/or prophylactic ingredients. Such carriers include liquids such as water, saline, glycerol, polyethyleneglycol, hyaluronic acid, ethanol, etc. Suitable carriers for nonliquid formulations are also known to those of skill in the art. Pharmaceutically acceptable salts can be used in the compositions of the present invention and include, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients and salts is available in *Remington's Pharmaceutical Sciences,* 18th Edition (Easton, Pennsylvania: Mack Publishing Company, 1990).

Additionally, auxiliary substances, such as wetting or emulsifying agents, biological buffering substances, surfactants, and the like, may be present in such carriers. A biological buffer can be virtually any solution which is pharmacologically acceptable and which provides the formulation with the desired pH, i.e., a pH in the physiologically acceptable range. Examples of buffer solutions include saline, phosphate buffered saline, Tris buffered saline, Hank's buffered saline, and the like.

Depending on the intended mode of administration, the pharmaceutical compositions may be in the form of solid, semi-solid or liquid dosage forms, such as, for example, tablets, suppositories, pills, capsules, powders, liquids, suspensions, creams, ointments, lotions or the like, preferably in unit dosage form suitable for single administration of a precise dosage. The compositions will include an effective amount of the selected drug in combination with a pharmaceutically acceptable carrier and, in addition, may include other pharmaceutical agents, adjuvants, diluents, buffers, etc.

For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like. Liquid pharmaceutically administrable compositions can, for example, be prepared by dissolving, dispersing, etc., an active compound as described herein and optional pharmaceutical adjuvants in an excipient, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example, see Remington's Pharmaceutical Sciences, referenced above.

For oral administration, the composition will generally take the form of a tablet, capsule, a softgel capsule or may be an aqueous or nonaqueous solution, suspension or syrup. Tablets and capsules are preferred oral administration forms. Tablets and capsules for oral use will generally include one or more commonly used carriers such as lactose and corn starch. Lubricating agents, such as magnesium stearate, are also typically added. When liquid suspensions are used, the active agent may be combined with emulsifying and suspending agents. If desired, flavoring, coloring and/or sweetening agents may be added as well. Other optional components for incorporation into an oral formulation herein include, but are not limited to, preservatives, suspending agents, thickening agents, and the like.

Parenteral formulations can be prepared in conventional forms, either as liquid solutions or suspensions, solid forms suitable for solubilization or suspension in liquid prior to injection, or as emulsions. Preferably, sterile injectable suspensions are formulated according to techniques known in the art using suitable carriers, dispersing or wetting agents and suspending agents. The sterile injectable formulation may also be a sterile injectable solution or a suspension in a nontoxic parenterally acceptable diluent or solvent. Among the acceptable vehicles and solvents that may be employed are water, Ringer's solution and isotonic sodium chloride solution. In addition, sterile, fixed oils, fatty esters or polyols are conventionally employed as solvents or suspending media. In addition, parenteral administration may involve the use of a slow release or sustained release system such that a constant level of dosage is maintained.

Alternatively, the pharmaceutical compositions of the invention may be administered in the form of suppositories for rectal administration. These can be prepared by mixing the agent with a suitable nonirritating excipient which is solid at room temperature but liquid at the rectal temperature and therefore will melt in the rectum to release the drug. Such materials include cocoa butter, beeswax and polyethylene glycols.

The pharmaceutical compositions of the invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, propellants such as fluorocarbons or nitrogen, and/or other conventional solubilizing or dispersing agents.

Preferred formulations for topical drug delivery are ointments and creams. Ointments are semisolid preparations which are typically based on petrolatum or other petroleum derivatives. Creams containing the selected active agent, are, as known in the art, viscous liquid or semisolid emulsions, either oil-in-water or water-in-oil. Cream bases are water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. The specific ointment or cream base to be used, as will be appreciated by those skilled in the art, is one that will provide for optimum drug delivery. As with other carriers or vehicles, an ointment base should be inert, stable, nonirritating and nonsensitizing.

Formulations for buccal administration include tablets, lozenges, gels and the like. Alternatively, buccal administration can be effected using a transmucosal delivery system as known to those skilled in the art. The compounds of the invention may also be delivered through the skin or muscosal tissue using conventional transdermal drug delivery systems, i.e., transdermal "patches" wherein the agent is typically contained within a laminated structure that serves as a drug delivery device to be affixed to the body surface. In such a structure, the drug composition is typically contained in a layer, or "reservoir," underlying an upper backing layer. The laminated device may contain a single reservoir, or it may contain multiple reservoirs. In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or gel reservoir, or may take some other form. The backing layer in these laminates, which serves as the upper surface of the device, functions as the primary structural element of the laminated structure and provides the device with much of its flexibility. The material selected for the backing layer should be substantially impermeable to the active agent and any other materials that are present.

A pharmaceutically or therapeutically effective amount of the composition will be delivered to the subject. The precise effective amount will vary from subject to subject and will depend upon the species, age, the subject's size and health, the nature and extent of the condition being treated, recommendations of the treating physician, and the therapeutics or combination of therapeutics selected for administration. Thus, the effective amount for a given situation can be determined by routine experimentation. For purposes of the present invention, generally a therapeutic amount will be in the range of about 0.05 mg/kg to about 40 mg/kg body weight, more preferably about 0.5 mg/kg to about 20 mg/kg, in at least one dose. In larger mammals the indicated daily dosage can be from about 1 mg to 100 mg, one or more times per day, more preferably in the range of about 10 mg to 50 mg. The subject may be administered as many doses as is required to reduce and/or alleviate the signs, symptoms, or causes of the disorder in question, or bring about any other desired alteration of a biological system.

The delivery of polynucleotides, e.g., for delivering 15-lipoxygenase antisense oligonucleotides, can be achieved using any of the formulations described above, or by using recombinant expression vectors, with or without carrier viruses or particles. Such methods are well known in the art. See, e.g., U.S. Patent Nos. 6,214,804; 6,147,055; 5,703,055; 5,589,466; 5,580,859; Slater et al. (1998) J. Allergy Clin. Immunol. 102:469-475. For example, delivery of polynucleotide sequences can be achieved using various viral vectors, including retrovirus and adeno-associated virus vectors. See, e.g., Miller A.D. (1990) Blood 76:271; and Uckert and Walther (1994) Pharmacol. Ther. 63:323-347. Vectors which can be utilized for antisense gene therapy include, but are not limited to, adenoviruses, herpes viruses, vaccinia, or, preferably, RNA viruses such as retroviruses. Other gene delivery mechanisms that can be used for delivery of polynucleotide sequences to target cells include colloidal dispersion and liposome-derived systems, artificial viral envelopes, and other systems available to one of skill in the art. See, e.g., Rossi, J.J. (1995) Br. Med. Bull. 51:217-225; Morris et al. (1997) Nucl. Acids Res. 25:2730-2736; and Boado et al. (1998) J. Pharm. Sci. 87:1308-1315. For example, delivery systems can make use of macromolecule complexes, nanocapsules, microspheres, beads, and lipid-based systems including oil-in-water emulsions, micelles, mixed micelles, and liposomes.

As discussed above, the pharmaceutical formulations may contain one or more active agents that effectively regulate calcium homeostasis. The additional active agent may be, but is not limited to, an estrogen, a calcitonin, a bisphosphonate (e.g. alendronate, residronate, zolendronate and ibandronate), vitamin D₃ or an analogue thereof, an androgen, a fluoride salt, a parathyroid hormone or an analogue thereof, or an IGF, agents that alter regulation of transcription of naturally occurring hormone regulators involved in bone metabolism, and combinations thereof. This additional active agent can be administered to the subject prior to, concurrently with or subsequently to administration of the 15-lipoxygenase inhibitor of this invention.

Another aspect of the present invention is based upon the discovery of a correlation between a polymorphism in a 15-lipoxygenase gene and bone mineral density wherein the presence of certain polymorphisms correlates with decreased bone mineral density. A further aspect of the discovery is that the genotype is correlated with a predisposition to osteoporosis. The invention is of advantage in that by screening for the presence of the genotype it is possible to identify individuals likely to have this genetic predisposition. Accordingly, a further aspect of the invention provides a method of therapy comprising screening an individual for a predisposition to bone loss and, if a predisposition is identified, treating that individual to delay or reduce or prevent bone loss.

According to the diagnostic and prognostic method of the present invention, alteration, including deletions, insertions and point mutations in the coding and noncoding regions, of the wild-type 15-lipoxygenase locus is detected. Thus, in mice, for example, alternations on chromosome 11 are preferably detected, whereas in humans, alternations on chromosome 17 are detected. In addition, the method can be performed by detecting the wild-type 15-lipoxygenase locus and confirming the lack of a predisposition to bone loss disorders at the 15-lipoxygenase locus. Such mutations may be present in individuals either with or without symptoms of bone loss. In addition, there may be differences in the drug response or prognosis of symptomatic individuals that carry mutations in 15-lipoxygenase locus compared to those that do not.

Accordingly, one aspect of the invention provides a method of diagnosis comprising determining the genotype of the 15-lipoxygenase gene. Typically, the method determines whether an individual is homozygous or heterozygous for 15-lipoxygenase gene polymorphisms. Typically, the method of the invention is carried out by *in vitro* analysis of cells of an individual to determine the genotype of that individual at the 15-lipoxygenase gene locus.

Useful diagnostic techniques include, but are not limited to, microarray analysis, fluorescent in situ hybridization (FISH), direct DNA sequencing, PFGE analysis, Southern blot analysis, single stranded conformation analysis (SSCA), RNase protection assay, allele-specific oligonucleotide (ASO) analysis, dot blot analysis and PCR-SSCP, as is well known in the art.

Predisposition to bone loss disease can be ascertained by testing any tissue of a subject, such as a human patient, for mutations of the 15-lipoxygenase gene. For example, a person who has inherited a germline 15-lipoxygenase mutation would be prone to develop bone loss disease. This can be determined by testing DNA from any tissue of the person's body. Most simply, blood can be drawn and DNA extracted from the cells of the blood. In addition, prenatal diagnosis can be accomplished by testing fetal cells, placental cells or amniotic cells for mutations of the 15-lipoxygenase gene. Alteration of a wild-type 15-lipoxygenase allele, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. Another approach is the single-stranded conformation polymorphism assay (SSCA), where the fragments with shifted mobility on SSCA gels are sequenced to determine the exact nature of the DNA sequence variation. Other approaches based on the detection of mismatches between the two complementary DNA strands include clamped denaturing gel electrophoresis (CDGE), heteroduplex analysis (HA) and chemical mismatch cleavage (CMC). Once a mutation is known, an allele specific detection approach such as allele specific oligonucleotide (ASO) hybridization can be utilized to rapidly screen large numbers of other samples for that same mutation (see, e.g., Saiki et al.,Proc. Nad. Acad. Sci. USA 86:6230-6234 (1989)).

Detection of point mutations may be accomplished by molecular cloning of the 15-lipoxygenase allele(s) and sequencing the allele(s) using techniques well known in the art. Alternatively, the gene sequences can be amplified directly from a genomic DNA preparation from the tissue, using known techniques. The DNA sequence of the amplified sequences can then be determined. The presence of an allele can be confirmed by single-stranded conformation analysis (SSCA), denaturing gradient gel electrophoresis (DGGE or CDGE), RNase protection assays, allele-specific oligonucleotides (ASOs), allele-specific PCR, or single nucleotide extension assays, as is well known in the art.

In the preferred method, alleles are identified by using microchip technology. In this technique, thousands of distinct oligonucleotide probes or genes can be synthesized (U.S. Patent No. 5,412,087 to McGall *et al*.) or spotted as an array (U.S. Patent No. 6,110,426 to Shalon *et al*.) on a silicon or glass chip. Nucleic acid to be analyzed is generally fluorescently labeled and hybridized to the probes on the chip. Other labels include ³²P and biotin. It is also possible to study nucleic acid-protein interactions using these microarrays. Using this technique, the presence of mutations in the 15-lipoxygenase gene is identified.

The presence of an altered (or a mutant) 15-lipoxygenase gene correlates to an increased risk of bone loss disease. In order to detect a 15-lipoxygenase gene mutation, a biological sample is prepared and analyzed for a difference between the sequence of the 15-lipoxygenase allele being analyzed and the sequence of the wild-type 15-lipoxygenase allele. The mutant alleles can then be sequenced to identify the specific mutation of the particular mutant allele. The mutations in the 15-lipoxygenase gene, specifically on chromosome 11 for mice and chromosome 17 for humans, are then used for the diagnostic and prognostic methods of the present invention.

Thus, the present invention also provides a method of diagnosis of predisposition to bone loss in a subject, the method comprising detecting a polymorphism on human chromosome 17, wherein the polymorphism is indicative of predisposition to bone loss. In a preferred embodiment, the detecting comprises genotyping. In a more preferred embodiment, the genotyping consists of microsatellite markers or one or more single nucleotide polymorphisms.

In another embodiment, the present invention provides a method of diagnosis of predisposition to bone loss in a subject, the method comprising detecting a polymorphism in the 15-lipoxygenase gene of the subject.

The present invention provides 15-lipoxygenase inhibitors for use as therapeutic active substances to prevent bone loss. It also provides compounds identified by the methods hereinbefore described. Furthermore, a pharmaceutical composition is provided, in particular for the prevention of bone loss, comprising a compound hereinbefore described and a carrier. A kit for the diagnosis of predisposition to bone loss comprising at least one oligonucleotide for the detection of a polymorphism in the 15-lipoxygenase gene that is associated with bone loss is also provided.

Finally, the compounds, methods, uses, composition and kit substantially as described herein, especially with reference to the foregoing examples are also claimed.

Figure 1 depicts the comparison of SNP-based genotyping of pooled DNA samples with microsatellite genotyping of individual DNA samples for detecting polymorphisms related to bone mineral density. The significance of each allele-frequency difference was calculated using the z-test and plotted as an LOD score for all chromosomes. Dashed line indicates an LOD score of 3.3 that was set as the threshold for genome-wide significance.

Figure 2 shows the quantitation of Alox15 gene expression in mouse kidney using whole kidney RNA, where gene expression was analyzed using microarrays.

Figure 3 shows quantitation of Alox15 gene expression in mouse primary osteoblasts in response to increasing concentrations of IL-4 administered *in vitro.*

Figure 4 shows the locations of the single nucleotide polymorphisms (SNPs) identified in the Alox15 gene in mice.

Figure 5 shows the quantitation of alkaline phosphatase activity in human mesenchymal stem cells (hMSC) in response to 15-lipoxygenase inhibitors Compound 1 or Compound 2 relative to solvent only (DMSO) control.

Figure 6 shows the quantitation of alkaline phosphatase activity in hMSC with Compound 3 or Compound 2 as 15-lipoxygenase inhibitors and DMSO or 1,25-Vitamin D₃ as the controls.

Figure 7 shows the quantitation of the total calcium content of hMSC with Compound 3 or Compound 2 as 15-lipoxygenase inhibitors and DMSO or 1,25-Vitamin D₃ as the controls.

Figure 8 shows the quantitation of alkaline phosphatase activity in hMSC cultured for 9 days with the 5-LO inhibitors Zileuton, AA-861, and Rev-5901 relative to solvent only (DMSO) control. The lack of a response is in contrast to the effects seen with inhibitors of 15-lipoxygenas

Figure 9 shows the quantitation of the total calcium content of hMSC cultured for 16 day with 5-LO inhibitors Zileuton, AA-861, and Rev-5901 relative to solvent only (DMSO) or 1,25-Vitamin D3 controls. The lack of a response is in contrast to the effects seen with inhibitors of 15 lipoxygenase.

Below are examples of specific embodiments for carrying out the present invention. The examples are offered for illustrative purposes only, and are not intended to limit the scope of the present invention in any way.

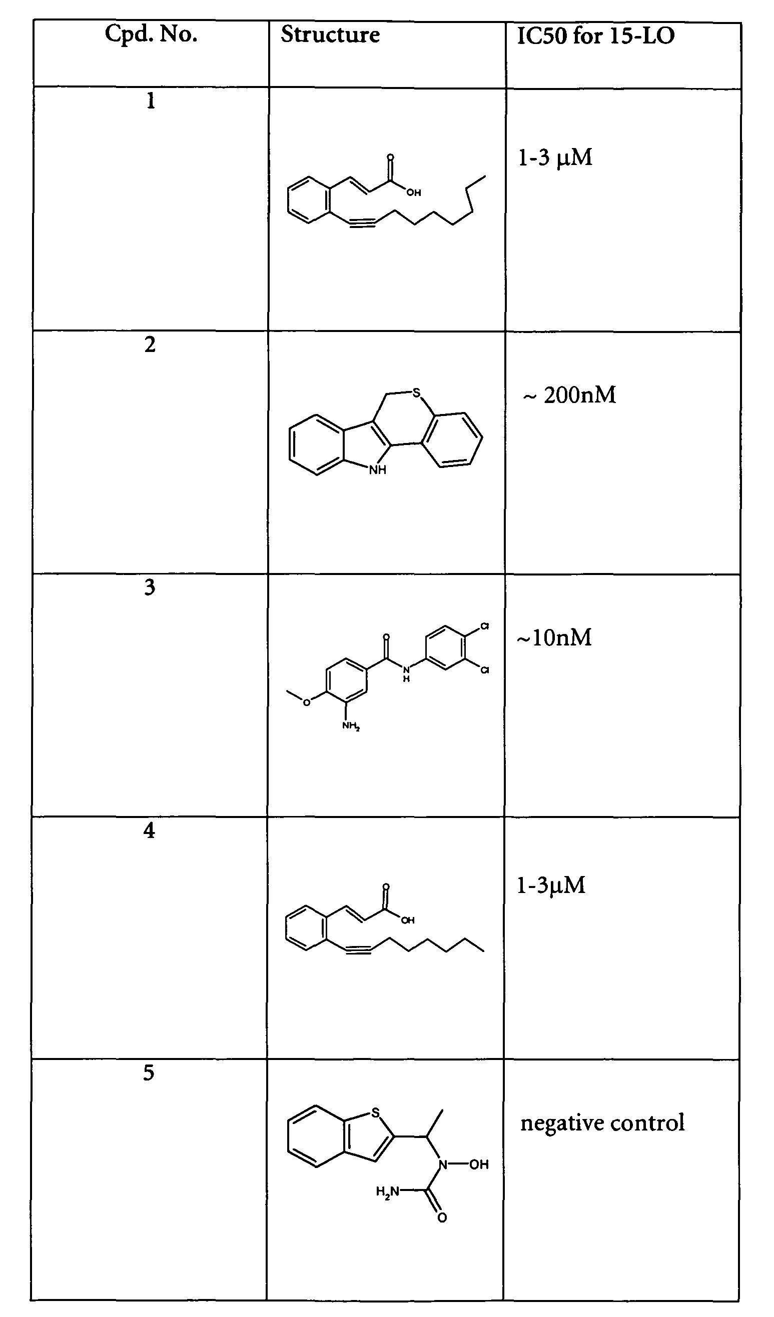

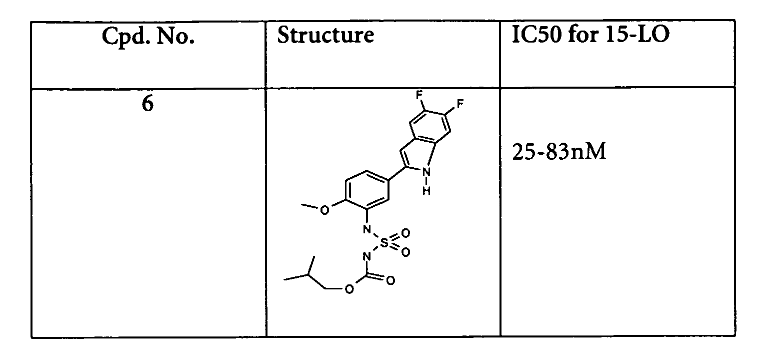

Compound 1, 3-(2-non-1-ynylphenyl)-propionic acid, is described in U.S. Patent No. 5,972,980.

Compound 2, 6,11-dihydro-5-thia-11-aza-benzo[a]fluorene, is described in WO97/12613.

Compound 3, 3-amino-N-(3,4-dichlorophenyl)-4-methoxybenzamide, is described in WO99/32433.

Compound 4, *trans*-3-(2-oct-1-ynyl-phenyl)-acrylic acid is described in U.S. Patent No. 4,713,486.

Compound 5, Zilueton, is described in U.S. Patent No. 4,873,259.

Compound 6, [[[5-(5,6-difluoro-1H-indol-2-yl)-2-methoxyphenyl]amino]sulfonyl]-carbamic acid-isobutyl ester, is described in WO01/96298.

Efforts have been made to ensure accuracy with respect to numbers used (e.g., amounts, temperatures, etc.), but some experimental error and deviation should, of course, be allowed for.

### Example 1

### SNP Genotyping Models.

Microsatellites (also called simple tandem repeat polymorphisms, or simple sequence length polymorphisms) constitute the most developed category of genetic markers. They include small arrays of tandem repeats of simple sequences (di-,tri-,tetranucleotide repeats) which exhibit a high degree of length polymorphism thereby providing a high level of information. Slightly more than 5,000 microsatellites easily typed by PCR-derived technologies, have been ordered along the human genome (Dib et al., Nature 380:152 (1996)).

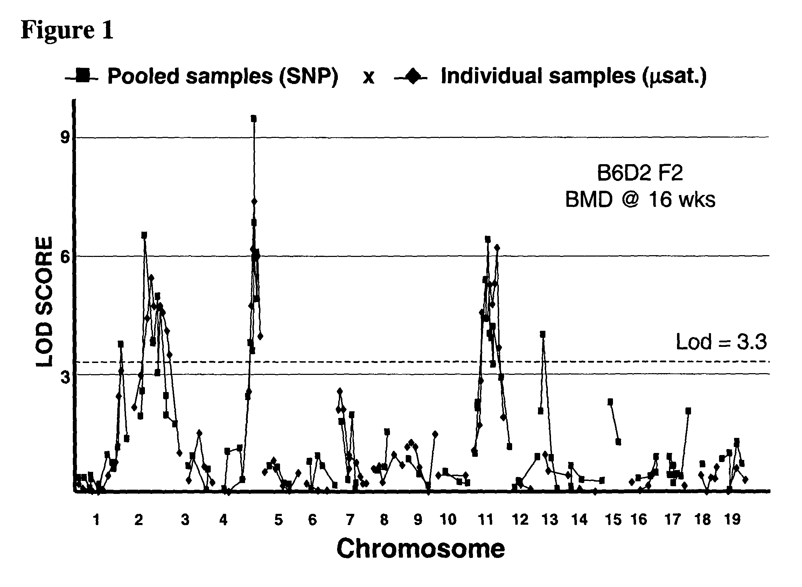

The method described by Grupe *et al*. (2001) Science 292: 1915-1918 was generally followed. To identify genetic factors regulating BMD, a genome scan was performed on 1000 F2 progeny of a C57BL/6 x DBA/2 intercross (Jackson Labs, Bar Harbor, Maine) at 16 weeks of age. The F2 progeny displayed a non-sex linked, normal distribution of BMD (Grupe et al., *Science*, 292, 1915-1918, (2001)). Phenotypically extreme F2 progeny with the highest (n=145) and lowest (n=149) BMD (top and bottom 15%) were subjected to a whole-genome-scan for association with BMD by genotyping individual DNA samples with 100 microsatellite markers. In addition, equal amounts of DNA from the high and low BMD F2 progeny were used to form two pools. Allele frequencies in the pooled samples were measured for 109 SNPs found in the mSNP database using the allele-specific kinetic PCR method. Differences in allele-frequency between the two extremes for each marker were scored. If a marker has no association with BMD, its expected frequency is 50% for both extremes. The significance of each allele-frequency difference was calculated using the z-test and plotted as a LOD score (Figure 1). A significant association (LOD score > 3.3) was found for four regions, located on chromosomes 1, 2, 4, and 11 by the microsatellite and SNP genotyping methods.

Thus, using the SNP genotyping method, candidate genes responsible for BMD were identified.

### Example 2

### Animal Models.

To identify the gene within the identified region on chromosome 11 which regulated BMD, gene expression differences between DBA/2, C57BL6/J (Jackson Labs, Bar Harbor, Maine) and a congenic mouse strain (D2.B6chr11, obtained from Oregon Health Sciences University) were analyzed using microarrays. The D2.B6chr11 congenic mouse had the C57BL/6J chromosome 11 interval from cM20 to cM50 introgressed onto the DBA2/J background. Microarrays, containing genes from the mouse genome, were hybridized with labeled cRNA obtained from whole kidneys, cultured primary osteoblasts and primary chondrocytes. Hybridizations were done with cRNA obtained from individual DBA/2J, C57BL/6J, and B6.D2chr11 congenic mice. Isolation of mRNA (2x poly(A) +), cRNA synthesis and hybridization were performed. Differential expression was assessed by pairwise comparison of DBA/2J and C57BL/6 mice, and by pairwise comparison of DBA/2J and B6.D2chr11 congenic mice.

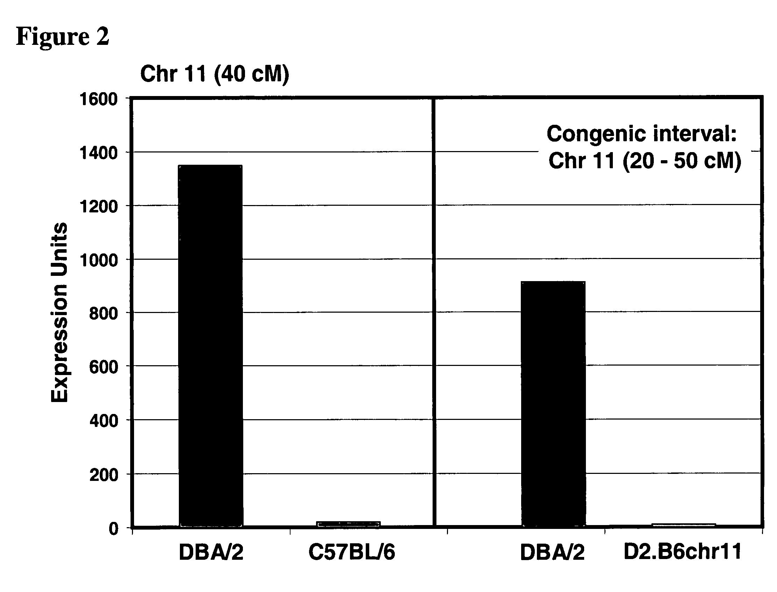

Differentially expressed genes, encoded within the interval (cM20-cM50) on chromosome 11 were selected for further characterization. A single gene (Alox15 at cM 40 on chr. 11) was substantially differentially expressed between the two strains examined (Figure 2). The DBA/2J strain had elevated levels of expression of Alox15 compared to D2.B6chr11 and C57BL/6J mice and also decreased BMD relative to both the other strains. Thus, elevated Alox15 expression in the DBA/2J mice contributed to a lowered bone mineral density.

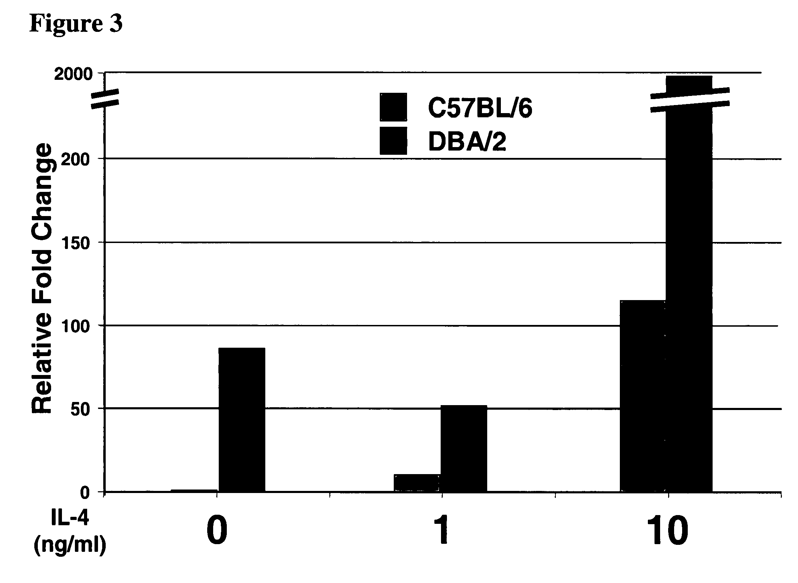

Differential Alox15 mRNA expression in osteoblast cells was assessed using real-time PCR. The cells were prepared from DBA/2J, C57BL/6J, and D2.B6chr11 mice (Figure 3). Total RNA was treated with DnaseI to remove contaminating genomic DNA. The RNA was heated at 65 °C for 10 min to inactivate the DnaseI. All PCR reactions were performed in a 100-µl volume. The RNA (100 ng) was subjected to RT PCR using rTh DNA polymerase (2 units) (Perkin Elmer) in a reaction mix containing 5X EZ buffer, Mn(OAc) (3 mM), ethidium bromide (1 µg/ul), dNTPs (200 µM), forward primer (200 nM), and reverse primer (200 nM). The cDNA was generated by incubating samples for 30 min at 60 °C. This was followed by PCR for 60 cycles (95 °C, 20 sec; 58 °C, 20 sec), and then incubated for 20 min at 72 °C. The fluorescence at each cycle is related to the amount of product generated and is measured using a kinetic thermocycler and analyzed using software provided (Germer and Higuchi, *Genome Research,* 9, 72-78, (1999)). In addition to the kidney, osteoblasts from DBA/2J mice also produced elevated basal levels of Alox15 m RNAcompared to C57BL/6J mice. Alox15 RNA was induced by IL-4 in the osteoblasts from C57BL/6 mice to a greater degree relative to DBA/2 mice (Figure 3).

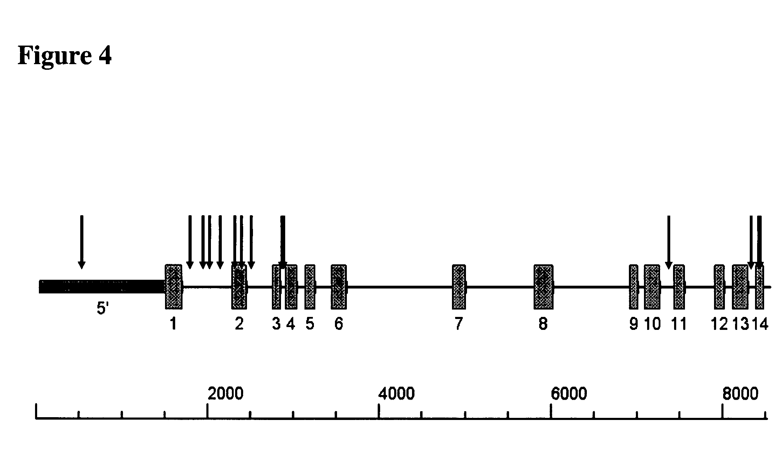

To identify a molecular basis for the strain-specific difference in ALox15 expression, the genomic DNA for the Alox15 gene from DBA/2J and C57BL6/J mice was sequenced using methods known in the art. Fourteen DNA sequence polymorphisms distinguishing the two strains were identified (diagramed in Figure 4 and in Table 1 below). The Ensembl gene designation for 15LO is ENSMUSG00000018924. The gene sequence, encompassing positions 70929619 to 70937482 of the mouse genome is given in Seq ID No. 1. The first SNP in Table 1, position 70938498 in the mouse genome sequence, is located at position 541 of the sequence in Genbank accession No. U04332, which is listed in Seq ID No. 2.

| Table 1. Alox15 SNPs identified in DBA/2J compared to C57BL/6J mice. | | | |
|---|---|---|---|
| **SNP Position in mouse genome (bp)** | **Nucleotide Change C57BL/6J_DBA2/J** | **Intron/Exon** | **A.A. Position** |
| 70938498 | C_G | ~ 1kb upstream of putative TATA box in 5' utr | |
| | | | |
| 70937181 | A_G | Intron 1 | |
| 70936977 | T_C | Intron 1 | |
| 70936975 | A_T | Intron 1 | |
| 70936918 | C_T | Intron 1 | |
| 70936336 | T_C | Exon 2 | 62/Phe(TTT) _ Phe(TTC) |
| | | | |
| 70936248 | G_A | Exon 2 | 91/Ser(TCG)_ Ser(TCA) |
| | | | |
| 70936136 | G_A | Intron 2 | |
| 70935775 | G_A | Intron 3 | |
| 70935765 | C_T | Intron 3 | |
| 70931174 | A_G | Intron 10 | |
| 70930258 | A_G | Intron 13 | |
| 70930156 | C_T | Exon 14 | 616/Pro(CCA) _ Ser(TCA) |
| | | | |
| 70930151 | C_T | Exon 14 | 617/Asn(AAC) _.Asn(AAT) |
| | | | |
| 70929999 | T deleted in DBA/2J | 11th base of 3' utr | |

### Example 3

### Ability of 15-lipoxygenase Inhibitors to Increase Bone Formation

In order to determine the ability of inhibitors of 15-lipoxygenase to stimulate bone formation and bone accretion, the ability of the 15-LO inhibitors to promote measures of differentiation of stem cells into bone forming cells (osteoblasts) was tested *in vitro.* In particular, the ability of three inhibitors, Compound 1 ( 3-(2-non-1-ynylphenyl)-propionic acid), Compound 2 (6,11-dihydro-5-thia-11-aza-benzo[a]fluorene), and Compound 3 (3-amino-N-(3,4-dichlorophenyl)-4-methoxybenzamide), prepared using literature procedures, to promote the differentiation of human mesenchymal stem cells into osteoblasts was determined by measuring two markers of osteoblast differentiation, cellular alkaline phosphatase activity and culture calcium content.

In general, human mesenchymal stem cells (hMSC, Poietics #PT-2501, BioWhittaker) were cultured in 12-well collagen coated culture plates at 3100 cells per square cm in 1 ml culture medium (Clonetics cat#PT-4105 plus osteoblast inducers (100nM Dex, 0.05mM L-Ascorbic acid-2-phosphate, 10 mM beta-glycerolphosphate). The three 15-LO inhibitors were added individually to each of the cultures, except for control wells to which was added 0.1% DMSO (negative control) or 1nM of 1,25-Vitamin D₃ (positive control). The concentrations of the inhibitors were as follows: Compound 1 at 3 and 30 µM; Compound 2 at 0.1, 0.2, 0.3, or 1.0µM and Compound 3 at 3, 10, or 30µM. Four to eight independent replicate cultures were prepared at each dose level. At the end of the experiments, the cultured cells were harvested by scraping the wells into the appropriate medium for further analysis of either alkaline phosphatase (Sigma kit#104-LL), or cellular calcium (Sigma kit#587-M). Cultures harvested for alkaline phosphatase assessment were harvested by scraping the cells and resuspending into 250µL Tris buffered 0.1% Triton X-100, and then assayed either fresh or following freeze-thaw. Separate cultures to be assayed for total calcium content were scraped and resuspended into 250µL 0.5M HCL. Results of these assays were normalized to total cellular DNA, thereby normalizing for differences in cell proliferation. At the same times that cultures were harvested for alkaline phosphatase and calcium, separate replicate cultures were harvested by scraping the cells and resuspending them in 250µL Hank's Balanced Salt Solution, and cellular number assessed by DNA (DNeasy Tissue Kit, Qiagen cat#69506).

### Quantitation of Osteoblast Differentiation in vitro.

For the quantitation of alkaline phosphatase activity, the inhibitors were prepared in 0.1% DMSO and added to the cultures on day 1 and every 2-3 days thereafter for 14-16 days. Three different sets of experiments were performed.

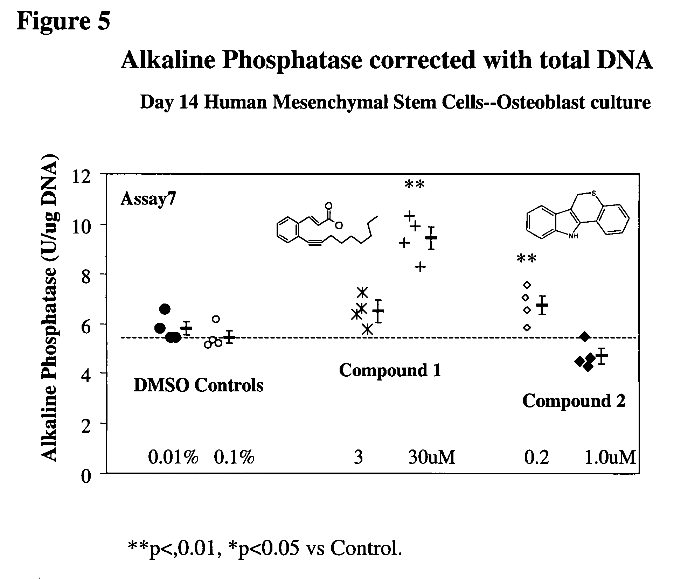

In the first experimental set, Compound 1 or Compound 2 (0.2µM or 1 µM) were used as inhibitors with the solvent (DMSO) as the negative control, and four independent replicate cultures were prepared at each dose level. The plates were cultured for 14 days. The DNA-normalized activity of alkaline phosphatase is plotted in Figure 5.

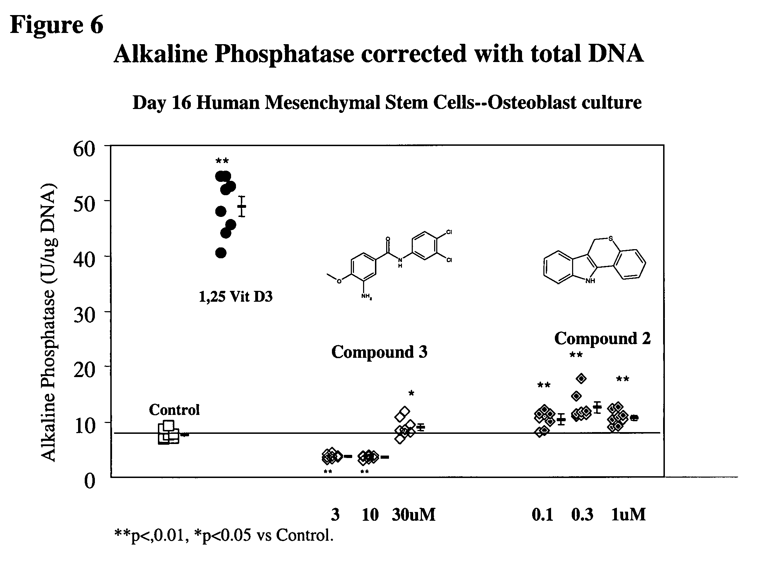

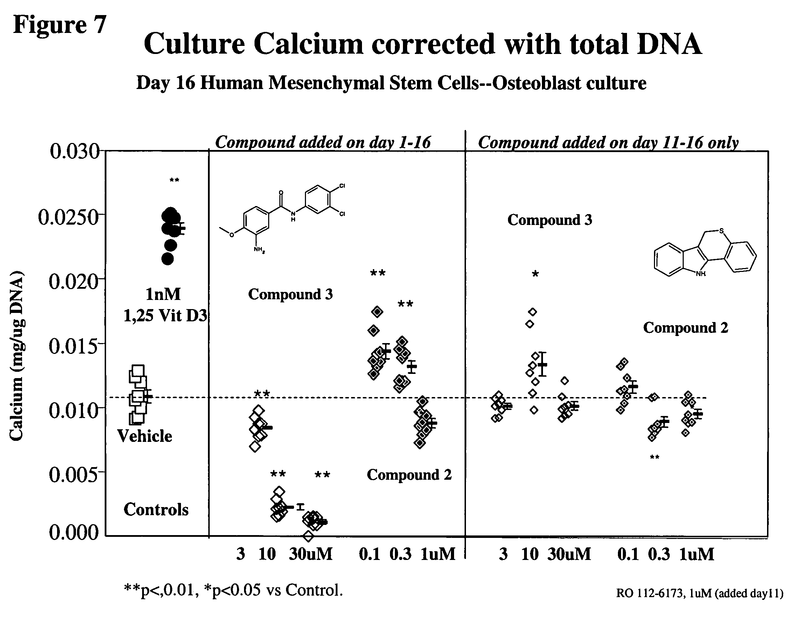

The second experimental set was comprised of eight replicate cultures with Compound 3 (3, 10, and 30µM) or Compound 2 (0.1, 0.3, 1.0µM) as the 15-LO inhibitors. Inhibitors were added either on Day 1 or Day 11. DMSO was used as the negative control, and 1,25-Vitamin D3 as the positive control for osteoblast differentiation induction. The normalized activity of alkaline phosphatase and calcium content is plotted in Figures 6 and 7, respectively. Compound 2 was most effective when added on Day 1, and Compound 3 was most effective when added on Day 11.

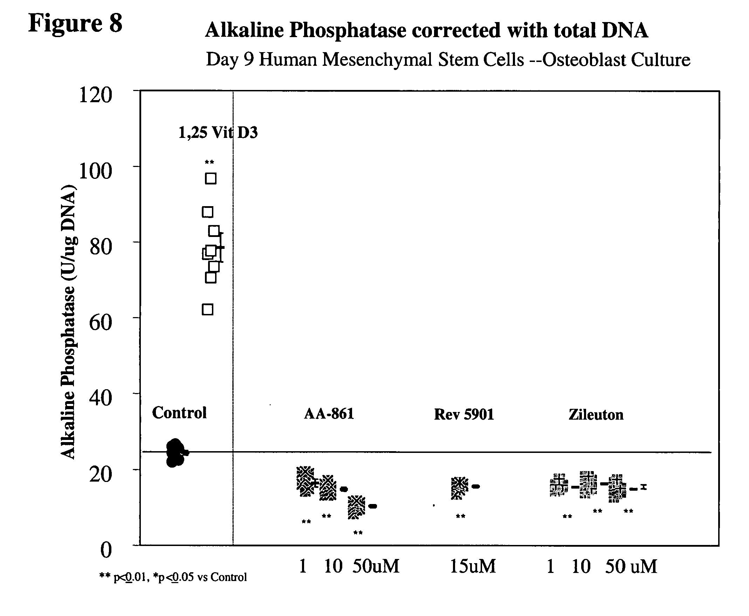

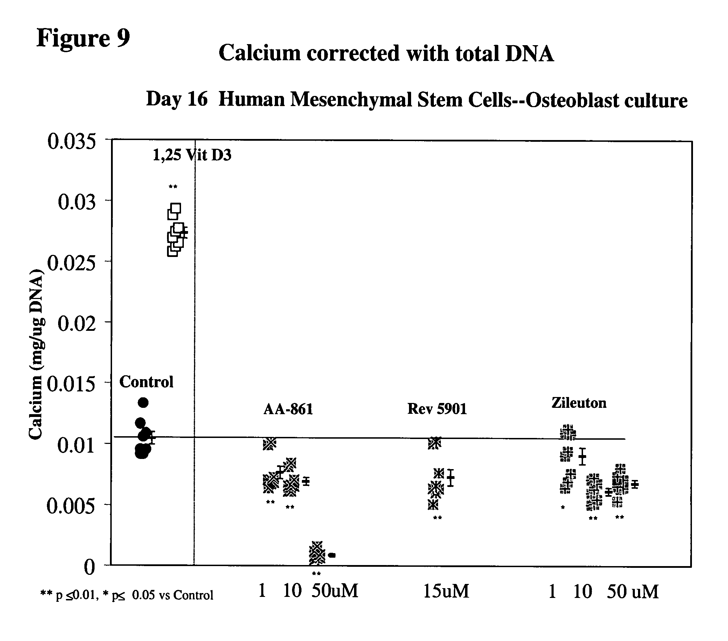

As a control for the specificity of 15-LO inhibition for the induction of osteoblast differentiation of stem cells in vitro, the third set of experiments studied eight replicates of standard lipoxygenase inhibitors recognized as more specific to the 5-LO enzyme, rather than the 15-LO enzyme. Zileuton (1,10, 50uM, prepared at Roche as) Compound 5, AA-861 (1, 10, 50uM, Sigma # A3711) and Rev-5901 (15uM, Sigma #R5523) were prepared fresh every 2-3 days and tested in the human osteoblast precursor cells. The 5-LO inhibitors were found to lack ability to induce alkaline phosphatase activity on day 9 or culture calcium deposition after 16 days of culture, as seen in Figures 8 and 9, respectively.

The results from the quantitation of markers of osteoblast differentiation, both alkaline phosphatase activity and culture calcium content, in hMSC cultures stimulated to differentiate into osteoblasts demonstrate that addition of specific 15-lioxygenase inhibitors promotes differentiation of the human bone forming cells *in vitro.*

In addition, the ability of 15-lipoxygenase inhibitors to stimulate bone formation and bone accretion is measured by the use of clonogenic assays where the clonogenic potential of the bone marrow precursor cells is measured. The osteoblast and osteoclast cells are treated with 15-lipoxygenase inhibitors. In one method, the cells and the inhibitors are incubated *in vitro.* In the another method, the mammal is administered the inhibitor, the bone marrow precursors are isolated, and then plated. For both methods, the colony forming units derived from these marrow cells is measured. One can screen for compounds with the potential for increasing bone mineral density if they are can be shown to increase the osteoblast clonogeneic potential of the marrow or decrease the osteoclast clonogeneic potential of the marrow.

### Example 4

### Disruption of the 15-Lipoxygenase gene results in increased femoral bone mineral density

Initially, 15-LO deficient mice were shown to have minor phenotypic changes, but subsequently it was shown that these mice are partially protected from atherosclerotic lesions in a mouse model (Sun and Funk, *J. Biol. Chem.,* 271, 24055-24062, (1996); Cyrus et al., *J. Clin. Invest*., 103, 1597-1604, (1999)). Moreover, BMD was not measured in 15-LO deficient mice. To examine the effect of genetic disruption of 15-LO on bone mineral density, 15-LO "knock-out" (15-LO-KO) mice were compared to parental mice with respect to bone mineral density. Bone mineral measurements were determined by dual energy X-ray absorptiometry (DEXA). All studies were performed with a Lunar PIXImus densitometer (Lunar Corp., Madison, WI). Whole body densitometric analyses were performed on anesthetized mice that were 4 months of age when the acquisition of adult bone mass is complete. The global window was defined as the whole body image minus the calvarium, mandible, and teeth. After sacrifice, the right femora were carefully removed and cleaned of adhering tissue prior to DEXA scanning. Whole body bone mineral density was not significantly changed between 15-LO-KO and C57BL/6 parental mice (49.8 +/- 0.6 mg/cm² vs. 49.5 +/- 0.4 mg/cm²). Importantly, however, femoral BMD was significantly increased in the 15-LO knock out mice compared to the C57BL/6 parental strain (54.0 +/- 1.2 mg/cm² vs. 49.7+/- 0.7 mg/cm² ). These results demonstrate that disruption of 15-LO leads to increased femoral bone mineral density.

### Example 5

### 15-Lipoxygenase Inhibitors promote bone formation in vivo

The ability of 15-lipoxygenase inhibitors to promote bone formation *in vivo* was assessed by measuring the ability of the inhibitors to improve bone parameters in a mouse model of osteoporosis. Constitutive expression of an Interleukin-4 transgene from the Lck gene promoter (Lck-IL4) in C57BL/6 mice results in a severe bone phenotype mimicking osteoporosis. (Lewis et al., *Proc. Natl. Acad. Sci.,* 90, 11618-22, (1993)) and these mice were used to demonstrate the effect of treatment with 15-LO inhibitors on bone mass.

The four experimental groups used are shown in Table 2. At the time of weaning, wild type C57BL/6 or transgenic Lck-IL4 mice received 150mg/kg bid daily of Compound 2 PD146176 in their food for 84 days (Diet 5001: 23% protein, 10% fat, 0.95% calcium, and 0.67% phosphorus; PMI Feeds, Inc., St. Louis, MO). Mice were permitted access to one-half the daily food intake twice daily at approximately 12 h intervals and diet intake was monitored daily. Water was available ad libitum. The control groups received the same diet without Compound 2. All groups consumed food equally well over the course of the experiment.

**Table 2.**

| C57BL/6 | | Lck-IL4 transgenic | |
|---|---|---|---|
| Control | Compound 2 | Control | Compound 2 |
| n=17 (8F/9M) | n=18 (9F/9M) | n=20 (11F/9M) | n=22 (11F/11M) |
| Table 2. Experimental groups used for in vivo inhibitor studies. After weaning (approximately 28 days), mice were fed chow with or without 15-LO inhibitor (Compound 2) for 84 days. F, female and M, male animals were equally represented among the groups. | | | |

The effects of IL4 overexpression and 15-LO inhibition on whole body parameters, femoral BMD and geometry, and femoral biomechanics are shown in Table 3. Overexpression of IL4 results in a significant decrease in whole body BMD, body weight, and hematocrit and an increase in percent body fat. Overexpression of IL4 also reduced femoral BMD, cortical area, moment of inertia, and cortical thickness compared to wild type C57BL/6 mice. The increase in marrow area in Lck-IL4 mice correlates with the decreased hematocrit and associated anemia that has been reported. Additionally, IL4 overexpression had a significant effect on femoral biomechanics, including decreased failure load, bone stiffness and bone strength (Table 3). The mice that were fed the 15-LO inhibitor (Compound 2) showed partial reversal of the deleterious effects on whole body and femoral bone (Table 3). The drug-treated mice had a significant increase in whole body BMD, hematocrit, femoral BMD, and cortical thickness relative to control mice carrying the Lck-IL4 transgene. Importantly, the 15-LO inhibitor treated mice had a significant increase in femoral biomechanics, including failure load, stiffness and strength. This is particularly relevant to osteoporosis in that the level of bone strength and failure load are significant determinants in patients that go on to develop fractures. In summary, the results indicate that inhibition of 15-LO *in vivo* leads to a higher BMD and higher bone strength in an animal model of osteoporosis.

**Table 3.**

| Whole Body | | | | |
|---|---|---|---|---|
| Phenotype | Effect of IL-4 overexpression | p value | Effect of IL-4 overexpression + Compound 2 | p value |
| Body Weight | -7% | 0.042 | NC | ns |
| % Body Fat | +19% | 0.007 | NC | ns |
| Hematocrit | -25% | 2 x 10⁻⁹ | +20% | 2 x 10-5 |
| Whole Body BMD | -11% | 2 x 10⁻⁹ | +5% | 7 x 10-5 |
| | | | | |

| Femoral BMD & Geometry | | | | |
|---|---|---|---|---|
| Phenotype | Effect of IL-4 overexpression | p value | Effect of IL-4 overexpression + Compound 2 | p value |
| BMD | -17% | 4 x 10⁻¹² | +9% | 3 x 10⁻⁶ |
| Length | NC | ns | NC | ns |
| Total area | NC | ns | -4% | 0.035 |
| Cortical area | -25% | 6 x 10⁻¹² | NC | ns |
| Marrow area | +16% | 4 x 10⁻⁷ | - 7% | 7 x 10⁻⁵ |
| Moment of Inertia | -17% | 7 x 10⁻⁵ | NC | ns |
| Cortical thickness | -27% | 9 x 10⁻¹⁰ | +6% | 0.037 |
| | | | | |

| Femoral Biomechanics | | | | |
|---|---|---|---|---|
| Phenotype | Effect of IL-4 overexpression | p value | Effect of IL-4 overexpression + | p value |
| | | | **Compound 2** | |
| Failure load | -45% | 6 x 10⁻¹³ | +18% | 0.003 |
| Stiffness | -48% | 3 x 10⁻¹¹ | +11% | 0.09 |
| Strength | -34% | 2 x 10⁻¹² | +22% | 2 x 10⁻⁴ |

Table 3. Effects of IL4 overexpression and 15-LO inhibitor on whole body and femoral bone parameters. Whole body and femoral bone parameters were measured in mice carrying the Lck-IL4 transgene that leads to overexpression of IL4 compared to C57BL/6 controls. These parameters were also measured in Lck-IL4 mice that were fed the 15-LO inhibitor Compound 2 compared to untreated Lck-IL4 controls. NC, Not Changed between groups. ns, p-value was not significant.

### Animals

All mice used in the *in vivo* experiments were bred under identical conditions at the Portland VA Veterinary Medical Unit from stock originally obtained from The Jackson Laboratory (Bar Harbor, ME). Breeding mice were maintained for no more than three generations from stock obtained from The Jackson Laboratory. At the time of weaning the mice were group housed (9-10 animals per cage) in a 12 hr light/dark cycle (6:00 AM to 6:00 PM) at 21 ± 2°C. All procedures were approved by the VA Institutional Animal Care and Use Committee and performed in accordance with National Institutes of Health guidelines for the care and use of animals in research.

### Bone densitometry.

Bone mineral measurements were determined by dual energy X-ray absorptiometry (DEXA). All studies were performed with a Lunar PIXImus densitometer (Lunar Corp., Madison, WI). Whole body densitometric analyses were performed on anesthetized mice that were 4 months of age when the acquisition of adult bone mass is complete. The global window was defined as the whole body image minus the calvarium, mandible, and teeth. After sacrifice, the right femora were carefully removed and cleaned of adhering tissue prior to DEXA scanning.

### Specimen Processing

Adult mice (16 weeks old) were euthanized by CO₂ inhalation and weighed to the nearest 0.1 gm. Cardiac blood draws were obtained immediately upon sacrifice and a small aliquot of whole blood was reserved for hematocrit determination. Lumbar vertebrae and both femora were immediately harvested, wrapped in sterile gauze soaked in phosphate-buffered saline, and stored frozen at less than about -20 °C for subsequent analyses.

### Femoral Shaft Geometry

The cross-sectional geometric parameters of the mid-diaphysis of the femora were determined using a portable x-ray microtomograph (Model 1074, Skyscan, Antwerp, Belgium). Scans were taken at 40mm intervals, and a slice located at the midpoint of the femur was analyzed for total area (FCSA), cortical area (Ct Ar) and medullary canal area (Ma Ar). In addition, using the pixels contained in the cortical region of the digital image, the radius from the centroid of the cross-section to the outer fiber in the anterior-posterior plane, the areal moment of inertia (Ixx) in the plane of bending and the average cortical thickness (Ct Th) were calculated.

### Biomechanical Studies

The femur was tested to failure in three-point bending on a high resolution materials test apparatus (Instron Model 4442, Canton, MA). Failure load and stiffness were determined using system software. Bending strength was then calculated using the cross-sectional areal measurements previously determined.

### Data Analysis

All data were analyzed by a two-way analysis of variance (ANOVA) using the JMP software package (SAS Institute).

### Example 6

### Ability of a 15-lipoxygenase Inhibitor to Increase Bone Formation In Vivo

In order to determine the ability of inhibitors of 15-lipoxygenase to stimulate bone formation and bone accretion, [[[5-(5,6-difluoro-1H-indol-2-yl)-2-methoxyphenyl]amino]sulfonyl]-carbamic acid-isobutyl ester, Compound 6, was prepared as described in WO0196298 ) and tested in the standard estrogen deficiency Rat OVX osteopenia assay.

Three month old rats are ovariectomized (Ovx) and administered 1mg/kg/day Compound 6 or 0.1µg/kg/day 1,25-dihydroxy vitamin D₃ (positive control, abbreviated Vit. D in Table) by oral gavage once a day starting at 2 weeks post-ovariectomy and continuing for 7 weeks. The dose was increased to 2 mg/kg/day and continued until 11 weeks. Control groups, both sham (rats that were not ovariectomized) and Ovx, received vehicle only. The bone mineral density of the spine and right hip was determined by using the High Resolution Software package on a QDR-4500 Bone Densitometer (Hologic, Walthan, MA). The animals were scanned by placing them in a supine position such that the right femur was perpendicular to the main body and the tibia was perpendicular to the femur. The bone mineral density (g mineral/cm²) for the compounds at the hip and spine are given in Table 4 below. Animals treated with the 15-LO inhibitor showed increased BMD in the spine at >3 weeks and the proximal femur at > 7 weeks. Results are shown in Table 4.

**Table 4.**

| Treatment duration | Surgery | Treatment | Dose | BMD | | |
|---|---|---|---|---|---|---|
| | | | µg/kg/day | Lumbar Spine | | Proximal Femur |
| | | | | L2-L4 | L5 | |
| 3 weeks | Sham | Vehicle | | nd | nd | nd |
| | Ovx | Vehicle | | 0.2224 | 0.2343 | 0.2737 |
| | Ovx | Vit D | 0.1 | nd | nd | nd |
| | Ovx | Cpd6 | 1000 | 0.2347 *(0.055)* | 0.2480 *(0.041)* | 0.2838 *(0.103)* |
| 7 weeks | Sham | Vehicle | | .2664 | 0.2841 | 0.3068 |
| | Ovx | Vehicle | | 0.2303 | 0.2387 | 0.2829 |
| | Ovx | Vit D | 0.1 | 0.2628 *(0.023)* | 0.2742 *(0.015)* | nd |
| | Ovx | Cpd 6 | 1000 | 0.2474 *(0.019)* | 0.2549 *(0.120)* | 0.2932 *(0.033)* |
| 11 weeks | Sham | Vehicle | | 0.2740 | 0.2895 | 0.3177 |
| | Ovx | Vehicle | | 0.2353 | 0.2525 | 0.2842 |
| | Ovx | Cpd 6 | 2000 | 0.2507 *(0.009)* | 0.2626 *(0.059)* | 0.2986 *(0.039)* |
| Values in parentheses are p values vs OVX control at the same timepoint. nd = no data | | | | | | |

### Example 7

### A Genetic Manipulation to Reduce 15-lipoxygenase Expression in Laboratory MiceResults in Improved Bone Mass and Strength

In order to examine the relationship between 15-lipoxygenase expression and bone development, a genetically heterogeneous F₂ population was constructed from two progenitor strains, B6.129S2-*Alox15*^{*tm1Fun*} (*Alox15* knockout or 15LOKO) and DBA/2 (D2), which were purchased from The Jackson Laboratory (Bar Harbor, ME). The B6.129S2-*Alox15*^{*tm1Fun*} (15LOKO) mice are homozygous for a targeted mutation in *Alox15* and therefore cannot express arachidonate 15-lipoxygenase. In contrast, D2 mice exhibit high levels of *Alox15.* 15LOKO-D2 F₁ mice were bred locally from Jackson Laboratory parental lines and intercrossed to generate a total of 292 15LOKO-D2 F₂ mice. At the time of weaning the mice were group housed (2-5 animals per cage) and maintained with *ad libitum* water and laboratory rodent chow (Diet 5001: 23% protein, 10% fat, 0.95% calcium, and 0.67% phosphorus; PMI Feeds, Inc., St. Louis, MO) in a 12 hr light/dark cycle (6:00 AM to 6:00 PM) at 21 ± 2°C.

All mice were studied at 4 months of age when the acquisition of adult bone mass is complete (28). Bone mineral measurements were performed with a pencil beam Hologic QDR 1500 densitometer (Hologic, Waltham, MA) that was calibrated daily with a hydroxyapatite phantom of the human lumbar spine. Analysis was performed using the mouse whole body software (version 3.2), kindly provided by the manufacturer (Hologic, Waltham, MA). Densitometric analysis was performed on anesthetized mice. Food was withheld the night prior to examination (to eliminate confounding effects of undigested rodent chow on bone mineral density assessment), and the mice were anesthetized by isoflurane inhalation. The animals were weighed to the nearest 0.1 gm and then underwent bone density scanning. Mice were euthanized by CO₂ inhalation and spleens and femora were removed aseptically and then immediately frozen for subsequent analyses. All procedures were approved by the VA Institutional Animal Care and Use Committee and performed in accordance with National Institutes of Health guidelines for the care and use of animals in research.

Femoral structure (mid-shaft cortical bone area & cortical thickness) was measured with a desktop x-ray microtomographic scanner (SkyScan Model 1074, Aartselaar, Belgium). The left femur was tested to failure in three-point bending with a high-resolution materials test apparatus (Model 4442, Instron Corp., Canton, MA). An extensometer (Model 2630-113, Instron Corp.) was attached and system software (Series IX for Windows 95, Instron Corp.) was used to displace the actuator at a strain rate of 0.5%/sec. until failure occurred. Load and displacement (measured using the extensometer) data were recorded and failure load (F) and stiffness (k, calculated from the linear portion of the load versus displacement curve) were determined using system software.

Genomic DNA was isolated from individual mouse spleens using a salting-out method. Mice were genotyped with a polymerase chain reaction (PCR) protocol suggested by The Jackson Laboratory (http://aretha.jax.org/pub-cgi/protocols/protocols.sh?objtype=protocol&protocol id=304) designed to generate different sized products for the wild type (266 bp) and mutant (172 bp) *Alox15* gene. Amplification was performed on a Perkin-Elmer 9700 thermocycler (Branchburg, New Jersey). PCR products were separated on 4% agarose gels and visualized with ethidium bromide staining.

The 15LOKO-D2 F₂ population was composed of approximately equal numbers of male (n=141) and female (n=151) mice and, as is shown in Table 5 below, the Alox15 genotype frequency conformed to Hardy-Weinberg Principle expectations with 70 (24%) homozygous knockout (no 15-LO allele) mice, 153 (52%) heterozygous (single 15-LO allele from D2) mice, and 69 (24%) homozygous D2 (both 15-LO alleles from D2) mice. Although, there was no difference in body weight, F₂ mice homozygous for the *Alox15* knockout exhibited significantly higher whole body BMD than that of the D2 homozygous or heterozygous mice (*p* = 0.006 by ANOVA). Furthermore, F₂ mice homozygous for the *Alox15* knockout exhibited increased amounts of femoral shaft cortical bone (cortical area and cortical thickness) and significantly improved structural competence as evidenced by increased failure load and stiffness measures (Table 6).

**Table 5 ·**

| **15LO Genotype Correlates with BMD in 15LOKO-D2 F**_{**2**} **Population** | | | | |
|---|---|---|---|---|
| | Number of 15LO D2 Alleles | | | ANOVA |
| | 0 | 1 | 2 | *p* value |
| No. of Mice | 69 | 127 | 70 | |
| Body Weight, g | 28.2 ± 0.61 | 27.5 ± 0.43 | 29.2 ± 0.64 | NS |
| BMD, g/cm² | 66.1 ± 0.33 | 64.9 ± 0.25 | 65.0 ± 0.31 | *p* = 0.006 |

**Table 6 ·**

| 15LO Genotype Correlates with Femoral Bone Mass and Strength in 15LOKO-D2 F₂ Population | | | | |
|---|---|---|---|---|
| | Number of 15LO D2 Alleles | | | |
| | 0 | 1 | 2 | *p* value |
| No. of Mice | 15 | 15 | 15 | |
| Body Weight, g | 23.3 ± 0.54 | 23.9 ± 0.45 | 23.7 ± 0.40 | NS |
| BMD, g/cm² | 65.6 ± 0.46 | 64.8 ± 0.95 | 63.4 ± 0.65 | *p* = 0.005 |
| Ct Ar, mm² | 0.76 ± 0.03 | 0.78 ± 0.03 | 0.70 ± 0.02 | *p* = 0.046 |
| Ct Th, mm | 0.193 ± 0.005 | 0.200 ± 0.006 | 0.176 ± 0.004 | *p* = 0.006 |
| Failure Load, N | 20.5 ± 0.8 | 20.3 ± 0.8 | 18.0 ± 0.6 | *p* = 0.013 |
| Stiffness, N/mm | 126.5 ± 4.6 | 108.7 ± 5.1 | 110.3 ± 3.3 | *p* = 0.004 |
| (BMD = Whole body BMD; Ct Ar = femoral shaft cortical area; Ct Th = femoral shaft cortical thickness) | | | | |

Thus, novel methods for treating or preventing bone loss, increasing bone mineral density, and enhancing bone formation and accretion are disclosed. Although preferred embodiments of the subject invention have been described in some detail, it is understood that obvious variations can be made without departing from the spirit and the scope of the invention as defined by the appended claims.

## Claims

1. A method of diagnosis of predisposition to bone loss in a subject, the method comprising detecting a polymorphism on human chromosome 17, wherein the polymorphism is indicative of predisposition to bone loss.

2. The method of claim 1, wherein the detecting comprises genotyping.

3. The method of claim 2, wherein the genotyping consists of microsatellite markers or one or more single nucleotide polymorphisms.

4. A method of monitoring the efficacy of the use of a 15-lipoxygenase inhibitor selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester in reducing bone loss associated with osteoporosis, osteoarthritis, Paget's disease, or periodontal diseases in a mammalian subject, comprising measuring the level of 15-lipoxygenase in the subject.

5. The method of claim 4, wherein said bone loss is associated with osteoporosis, Paget's disease, or periodontal diseases.

6. The method of claim 5, wherein said bone loss is associated with osteoporosis.

7. The method of claims 4 to 6, wherein an additional active agent is used.

8. The method of claim 7, wherein the additional active agent is effective to regulate calcium resorption from bone.

9. The method of claim 8, wherein the additional active agent is selected from a group consisting of an estrogen, a calcitonin, a bisphosphonate, and an IGF.

10. A method of monitoring the efficacy of the use of a 15-lipoxygenase inhibitor selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester in increasing bone mineral density in a mammalian subject, comprising measuring the level of 15-lipoxygenase in the subject.

11. A method of monitoring the efficacy of the use of a 15-lipoxygenase inhibitor selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester in reducing the incidence of fracture in a mammalian subject, comprising measuring the level of 15-lipoxygenase in the subject.

12. A method of monitoring the efficacy of the use of a 15-lipoxygenase inhibitor selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester in healing fractures in a mammalian subject, comprising measuring the level of 15-lipoxygenase in the subject.

13. A method of monitoring the efficacy of the use of a 15-lipoxygenase inhibitor selected from the group consisting of 3-(2-non-1-ynylphenyl)-propionic acid, 6,11-dihydro-5-thia-11-aza-benzo[a]fluorene, 3-amino-N-(3,4-dichlorophenyl)-4-methoxybenzamide, *trans*-3-(2-oct-1-ynyl-phenyl)-acrylic acid and [[[5-(5,6-difluoro-1H-indol-2-yl)-2-methoxyphenyl]amino]sulfonyl]-carbamic acid-isobutyl ester in the treatment of osteoporosis in a mammalian subject, comprising measuring the level of 15-lipoxygenase in the subject.

14. A method of diagnosis of predisposition to bone loss in a subject, the method comprising detecting a polymorphism in the 15-lipoxygenase gene of the subject.
